# EUROPEAN PATENT SPECIFICATION

(11) **EP 0 557 469 B1**
(45) Date of publication and mention of the grant of the patent: **17.03.2004**
(21) Application number: 92913220.7
(22) Date of filing: 21.05.1992
(51) Int. Cl.: C12N 15/82, C12N 15/55, C12N 5/10, A23D 7/00, A23D 9/00, A01H 5/10, C11B 1/00, C12P 7/64, C12N 9/16

(54) **PLANT MEDIUM-CHAIN THIOESTERASES**
PFLANZLICHE MITTELKETTIGE THIOESTERASEN
THIOESTERASES A CHAINE MOYENNE D'ORIGINE VEGETALE

(30) Priority: 21.05.1991 US 704861; 07.10.1991 US 773096; 24.10.1991 US 782263; 22.01.1992 US 824247
(43) Date of publication of application: 01.09.1993
(73) Proprietor: Calgene LLC, Davis, California 95616 (US)
(72) Inventor: VOELKER, Toni, Alois, Davis, CA 95616 (US); DAVIES, Huw, Maelor, Davis, CA 95616 (US)
(74) Representative: Bosch, Henry
(86) International application number: PCT/US1992/004332
(87) International publication number: WO 1992/020236

(56) References cited:
- Archives of Biochemistry and Biophysics, Volume 290(1), issued October 1991, DAVIES et al., "Developmental Induction, Purfication, and further characterization of 12:0-ACP Thioesterase from immature cotyledons of Umbellularia Californica", pages 37-45, see the entire document.
- The Journal of Biological Chemistry, Volume 260(29), issued 15 December 1985, POULOSE et al., "Cloning and sequencing of the cDNA for S-acyl fatty acid synthase thioesterase from the uropygial gland of mallard duck", pages 15953-15958, see the entire document.
- Biochem. J., Volume 243, issued 1987, NAGGERT et al., "Cloning and Sequencing of the medium-chain S-acyl Fatty acid synthetase thioester hydrolase cDNA from rat mammary gland", pages 597-601, see the entire document.
- The Metabolism, Structure and Function of Plant Lipids, issued 1987, POLLARD et al., "Fatty Acid Synthesis in developing oilseeds", pages 455-463, see especially pages 459-460.
- Archives of Biochemistry and Biophysics, Volume 284(2), issued 01 February 1991, POLLARD et al., "A specific acyl-ACP thioesterase implicated in medium-chain fatty acid production in immature cotyledons of Umbellularia Californica", pages 306-312.
- Tibtech, Volume 5, issued February 1987, KNAUF, "The application of genetic engineering to oilseed crops", pages 40-47, see especially pages 44-45.
- Bio/Technology, Volume 6, issued October 1988, BAYLEY et al., "Metabolic Consequences of expression of the medium chain hydrolase gene of the rat in mouse NIH 3T3 cells", pages 1219-1221, see the entire document.
- CHEMICAL ABSTRACTS, Volume 112, issued 18 June 1990, DAULATABAD et al., "Studies on Verbenaceac seed oils", page 345, Abstract 232551q, see the entire document.
- Plant Physiology, Volume 84, issued 1987, CAO et al., "Acyl coenzyme A preference of diacylglycerol acyltransferase from the maturing seeds of Cuphea, maize, rapeseed, and canola", pages 762-765, see the entire document.
- BROWSE ET AL: 'Fatty acid composition of leaf lipids determined after combined digestion and fatty acid methyl ester formation from fresh tissue' ANALYTICAL BIOCHEMISTRY vol. 152, 1986, pages 141 - 145

## Description

### Background

Members of several plant familes synthesize large amount of predominantly medium-chain (C8-C14) triacylglycerols in specialized storage tissues, some of which are harvested for production of important dietary or industrial medium-chain fatty acids (F.D. Gunstone, The *Lipid Handbook* (Chapman & Hall, New York, 1986) pp. 55-112). Laurate (C12:0), for example, is currently extracted from seeds of tropical trees at a rate approaching one million tons annually (Battey, *et al*., *Tibtech* (1989) 71:122-125).

The mechanism by which the ubiquitous long-chain fatty acid synthesis is switched to specialized medium-chain production has been the subject of speculation for many years (Harwood, *Ann*. *Rev*. *Plant Physiol*. *Plant Mol*. *Biology* (1988) *39*:101-138). Recently, Pollard, *et al*., (*Arch*. *of Biochem*. *and Biophys.* (1991) *284*:1-7) identified a medium-chain acyl-ACP thioesterase activity in developing oilseeds of California bay, *Umbellularia californica*. This activity appears only when the developing cotyledons become committed to the near-exclusive production of triglycerides with lauroyl (12:0) and caproyl (10:0) fatty acids. This work presented the first evidence for a mechanism for medium-chain fatty acid synthesis in plants: During elongation the fatty acids remain esterified to acyl-carrier protein (ACP). If the thioester is hydrolized prematurely, elongation is terminated by release of the medium-chain fatty acid. The Bay thioesterase was subsequently purified by Davies *et al*., *(Arch. Biochem. Biophys*. (1991) *290*:37-45) which allowed the cloning of a corresponding cDNA and described it use to obtain related clones and to modify the triglyceride composition of plants (WO 91/16421).

### Summary of the Invention

By this invention, further properties and uses of plant medium-chain thioesterases are provided.

In a first embodiment, this invention relates to plant seed, which normally do not contain laurate, but now are found to contain laurate. Seed having as little as 1.0 percent mole laurate are significantly different from wild-type plant species which do not naturally store laurate in seed triglyceride oils. Seed having a minimum of about 15 percent mole laurate, 33 percent laurate or 50 percent laurate are contemplated hereunder. *Brassica* seed derived from such seed containing greater than 1.0 percent mole laurate is especially preferred.

In yet a different embodiment, this invention relates to a particular medium-chain thioesterase sequence, the Bay medium-chain thioesterase DNA sequence and to DNA constructs for the expression of this enzyme in a host cell. In particular, a start site for the structural gene sequence upstream to the start site previously reported for this sequence is described.

Other aspects of this invention relate to methods for using a plant medium-chain thioesterase. Expression of a plant medium-chain thioesterase in a bacterial cell to produce medium-chain fatty acids is provided. By this method, quantities of such fatty acids may be harvested in crystalline form from bacteria. Exemplified in the application is the use of *E.coli* and Bay thioesterase; the *fad* D *E*.*coli* mutant is particularly preferred. In addition, temperature ranges for improved laurate production are described.

Methods to produce an unsaturated medium-chain thioesterase by the use of a plant medium-chain thioesterase are also described herein. It is now found that, even in plants which exclusively produce and incorporate quantities of saturated medium-chain acyl-ACP fatty acids into triglycerides, the thioesterase may have activity against unstaturated fatty acids of the same length.

### Description of the Figures

Figure 1. The full length of a bay thioesterase (pCGN3822) having an ATG codon at nucleotides 145-147 is given. In 1A the nucleic acid sequence is given. In 1B, the translated amino acid sequence beginning at the ATG codon at nucleotides 145-147 is given.
Figure 2. Correlation of lauroyl thioesterase activity with the accumulation of acyl 12:0 in seeds of A thaliana is provided. Thioesterase activity is measured in developing seeds of different independent transgenic plants. The % 12:0 value reflects the percent lauroyl acyl group in total fatty acid extracts, as measured by quantitative gas chromatography.
Figure 3. Nucleic acid and translated amino acid sequence of a bay thioesterase clone, Bay D, which represents a second class of bay thioesterase genes, is presented.
Figure 4. Nucleic acid and translated amino acid sequences of two safflower thioesterase clones, pCGN3264 (4A) and pCGN3265 (4B), is presented. DNA sequence, including additional 3' untranslated sequence of pCGN3265 is presented in Figure 4C.
Figure 5. Nucleic acid sequence of a camphor thioesterase PCR fragment is presented in Figure 5A. Nucleic acid and translated amino acid sequences of a camphor PCR-generated thioesterase encoding sequence is presented in Figure 5B.
Figure 6. Nucleic acid sequence of a *Brassica campestris* thioesterase clone is presented in Figure 6. Translated amino acid sequence from the proposed MET initiation codon is also shown.
Figure 7. Lauroyl levels and C12:0-ACP thioesterase activity for seeds from transgenic *B. napus* is presented.
Figure 8. Comparison of safflower and bay thioesterase amino acid sequence is presented. The top line represents amino acids 61-385 of the safflower thioesterase amino acid sequence in Figure 4B. The bottom line represents amino acids 84-382 of the bay thioesterase amino acid sequence in Figure 1B.
Figure 9. Fatty acid composition of 100 seeds from transgenic *Arabidopsis* plant 3828-13 is compared to the fatty acid composition of seeds from a control *Arabidopsis* plant.
Figure 10. Fatty acid content of 26 transgenic *Arabidopsis* plants is provided in Figure 10A in order of increasing fatty acid content. The transformants producing detectable levels of laurate are indicated. In Figure 10B, the content of C18:3, C18:2 and C16:0 fatty acids in these plants are shown.
Figure 11. Mole percent laurate contents in developing seeds of transgenic *Brassica napus* are presented as the number of transgenic events yielding the indicated laurate levels. Results from pCGN3824 transformants are shown in Figure 11A and results from pCGN3828 transformants are shown in Figure 11B.
Figure 12. DNA sequence of a PCR fragment of a *Cuphea* thioesterase gene is presented. Translated amino acid sequence in the region corresponding to the *Cuphea* thioesterase gene is also shown.

### DETAILED DESCRIPTION OF THE INVENTION

Plant thioesterases, including medium-chain plant thioesterases are described in WO 91/16421 (PCT/US91/02960) and USSN 07/824,247.

A plant medium-chain thioesterase of this invention includes any sequence of amino acids, peptide, polypeptide or protein obtainable from a plant source which demonstrates the ability to catalyze the production of free fatty acid(s) from C8-C14 fatty acyl-ACP substrates under plant enzyme reactive conditions. By "enzyme reactive conditions" is meant that any necessary conditions are available in an environment (i.e., such factors as temperature, pH, lack of inhibiting substances) which will permit the enzyme to function.

Plant thioesterases are obtainable from the specific exemplified sequences provided herein and from related sources. For example, several species in the genus *Cuphea* accumulate triglycerides containing medium-chain fatty acids in their seeds, e.g., *procumbens, lutea, hookeriana, hyssopifolia, wrightii* and *inflata.* Another natural plant source of medium-chain fatty acids are seeds of the *Lauraceae* family: e.g., Pisa (*Actinodophne hookeri*) and Sweet Bay (*Laurus nobilis*). Other plant sources include *Ulmaceae* (elm), *Myristicaceae, Simarubaceae, Vochysiaceae,* and *Salvadoraceae,* and rainforest species of *Erisma, Picramnia* and *Virola*, which have been reported to accumulate C14 fatty acids.

As noted above, plants having significant presence of medium-chain fatty acids therein are preferred candidates to obtain naturally-derived medium-chain preferring plant thioesterases. However, it should also be recognized that other plant sources which do not have a significant presence of medium-chain fatty acids may be readily screened as other enzyme sources. In addition, a comparison between endogenous medium-chain preferring plant thioesterases and between longer and/or shorter chain preferring plant thioesterases may yield insights for protein modeling or other modifications to create synthetic medium-chain preferring plant thioesterases as well as discussed above.

One skilled in the art will readily recognize that antibody preparations, nucleic acid probes (DNA and RNA) and the like may be prepared and used to screen and recover "homologous" or "related" thioesterases from a variety of plant sources. For immunological screening methods, antibody preparations either monoclonal or polyclonal are utilized. For detection, the antibody is labeled using radioactivity or any one of a variety of second antibody/enzyme conjugate systems that are commercially available. Examples of some of the available antibody detection systems are described by Oberfilder *(Focus* (1989) BRL Life Technologies, Inc., *11*:1-5).

Homologous sequences are found when there is an identity of sequence, which may be determined upon comparison of sequence information, nucleic acid or amino acid, or through hybridization reactions between a known thioesterase and a candidate source. Conservative changes, such as Glu/Asp, Val/Ile, Ser/Thr, Arg/Lys and Gln/Asn may also be considered in determining amino acid sequence homology. Amino acid sequences are considered homologous by as little as 25% sequence identity between the two complete mature proteins. (*See* generally, Doolittle, R.F., *OF URFS* and ORFS (University Science Books, CA, 1986.) Typically, a lengthy nucleic acid sequence may show as little as 50-60% sequence identity, and more preferably at least about 70% sequence identity, between the target sequence and the given plant thioesterase of interest excluding any deletions which may be present, and still be considered related.

A genomic or other appropriate library prepared from the candidate plant source of interest may be probed with conserved sequences from plant thioesterase to identify homologously related sequences. Shorter probes are often particularly useful for polymerase chain reactions (PCR), especially when highly conserved sequences can be identified.

When longer nucleic acid fragments are employed (>100 bp) as probes, especially when using complete or large cDNA sequences, one would screen with low stringencies (for example 40-50°C below the melting temperature of the probe) in order to obtain signal from the target sample with 20-50% deviation, i.e., homologous sequences. (*See*, Beltz, *et al*. Methods *in Enzymology* (1983) *100*:266-285.).

Using methods known to those of ordinary skill in the art, a DNA sequence encoding a plant medium-chain thioesterase canbe inserted into constructs which can be introduced into a host cell of choice for expression of the enzyme, including plant cells for the production of transgenic plants. Thus, potential host cells include both prokaryotic and eukaryotic cells. A host cell may be unicellular or found in a multicellar differentiated or undifferentiated organism depending upon the intended use. Cells of this invention may be distinguished by having a plant thioesterase foreign to the wild-type cell present therein, for example, by having a recombinant nucleic acid construct encoding a plant thioesterase therein.

Also, depending upon the host, the regulatory regions will vary, including regions from viral, plasmid or chromosomal genes, or the like. For expression in prokaryotic or eukaryotic microorganisms, particularly unicellular hosts, a wide variety of constitutive or regulatable promoters may be employed. Among transcriptional initiation regions which have been described are regions from bacterial and yeast hosts, such as *E. coli, B. subtilis, Sacchromyces cerevisiae,* including genes such as beta-galactosidase, T7 polymerase, tryptophan E and the like.

For the most part, when expression in a plant host cell is desired, the constructs will involve regulatory regions (promoters and termination regions) functional in plants. The open reading frame, coding for the plant thioesterase or functional fragment thereof will be joined at its 5' end to a transcription initiation regulatory region such as the wild-type sequence naturally found 5' upstream to the thioesterase structural gene. Numerous other transcription initiation regions are available which provide for a wide variety of constitutive or regulatable, e.g., inducible, transcription of the structural gene functions. Among transcriptional initiation regions used for plants are such regions associated with the structural genes such as for CaMV 35S and nopaline and mannopine synthases, or with napin, ACP promoters and the like. The transcription/translation initiation regions corresponding to such structural genes are found immediately 5' upstream to the respective start codons. If a particular promoter is desired, such as a promoter native to the plant host of interest or a modified promoter, i.e., having transcription initiation regions derived from one gene source and translation initiation regions derived from a different gene source, including the sequence encoding the plant thioesterase of interest, or enhanced promoters, such as double 35S CaMV promoters, the sequences may be joined together using standard techniques. For most applications desiring the expression of medium-chain thioesterases in plants, the use of seed specific promoters are preferred. It is now observed that such a plant medium-chain thioesterase is biologically active when expressed in bacteria and heterologous plant cells.

In particular, it is now seen that plant seed which would not normally contain medium-chain fatty acid, either as free fatty acids or incorporated into triglyceride molecules, can be found to contain such medium-chain fatty acids. By seed which would not normally contain medium-chain fatty acid is meant seed which contains less than 0.1 mole percent of a given medium-chain fatty acid in total fatty acids. Thus, any plant seed containing a minimum of 1.0 mole percent of a given medium-chain fatty acid in total fatty acids is significantly modified. The use of a "mole percent in total fatty acids" is used to describe the relative ratio of medium-chain fatty acids out of the total fatty acid content. These figures can be converted to weight percent if desired.

Medium chain fatty acid contents from a minimum of 1.0 mole percent laurate in total fatty acids to a minimum of 50.0 mole percent laurate in total fatty acids have been measured. The total fatty acids of a plant seed include the embryo, endosperm and seed coat lipids. Additionally, it is noted that in medium-chain fatty acid containing seed, the content of laurate in total fatty acids directly corresponded with the laurate contents of the triacylglyceride. Thus, it is appropriate to consider the total fatty acid content as the "total extractable oils" as well.

As to triacylglycerides which incorporate the medium-chain fatty acids, it is not clear which positions of the glycerol backbone are involved. Based upon the high levels of medium-chain fatty acids measured, however, it is apparent that at least two positions of the triacylglyceride are involved.

Medium chain containing seed of Arabidopsis and *Brassica* are exemplified herein. In particular, seed of transgenic *Arabidposis* and *Brassica* plants containing novel fatty acid compositions as the result of expression of a heterologous medium-chain thioestesterase structural gene under the regulatory control of seed specific promoters are described. By the expression of the DNA sequence encoding the medium-chain thioesterase obtained from *Umbullaria californica* (Bay), laurate is now found in the extractible oil of these respective seeds. As the presence of laurate increases, a corresponding decrease in oleic acid (18:1) is observed. Other fatty acid compositional changes with increased laurate include the increase of myristate (14:0) and to a lesser degree, declines in the amounts of linolate (18:2), linolenate (18:3) and palmitate (16:0).

In *Arabidopsis*, analysis of 100 seed pools led to identification of transformed plants whose seeds contain up to 23.5 mole percent laurate, as compared to the approximately 0% laurate measured in control seeds. As the T2 seeds, that is mature seeds from T1 plants (original transformant) represent a segregating population, even higher levels of laurate would be expected in seeds from second generation plants (T2) grown from the T2 seed.

Analysis of transgenic *Brassica* seed expressing a bay thioesterase gene (25-30 seed pools) results in identification of transformants whose seeds contain up to 37 mole percent laurate Single and half-seed TAG analyses of these plants demonstrate that the levels of laurate in the segregating seed population are at least as high as 50 mole percent. Half-seed TAG analysis allows for identification of the highest laurate producing T2 seeds, and subsequent germination of the remaining seed portion to produce second generation plants with desirable high laurate seeds.

Correlations between the mole percent medium-chain fatty acid in total fatty acid and gene copy number have been observed. Therefore, although the minumim mole percent medium-chain fatty acid in total fatty acid measured is approximately 50.0 mole percent, it is possible to increase medium-chain fatty acid levels further by the insertion of more genes. Such techniques may involve genetic engineering or plant breeding methods.

Some genetic engineering approaches to increase medium-chain fatty acids would include insertion of additional DNA sequence encoding plant thioesterase structural genes into cells, use of transriptional initiation regions evidencing higher mRNA copy numbers or an improved timing specificity profile which corresponds better to the availability of substrate, for example. For example, analysis of the time course of laurate production, under regulatory control of a napin promoter, in seeds of a Brassica plant demonstrates that the appearance of medium-chain trioesterase activity lags behind the onset of storage oil synthesis by approximately 5-7 days. Calculations show that about 20% of the total fatty acids are already synthesized before the medium-chain thioesterase makes significant impact. Thus, substantially higher laurate levels (10-20%) might be obtained if the thioesterase gene is expressed at an earlier stage of embryo development

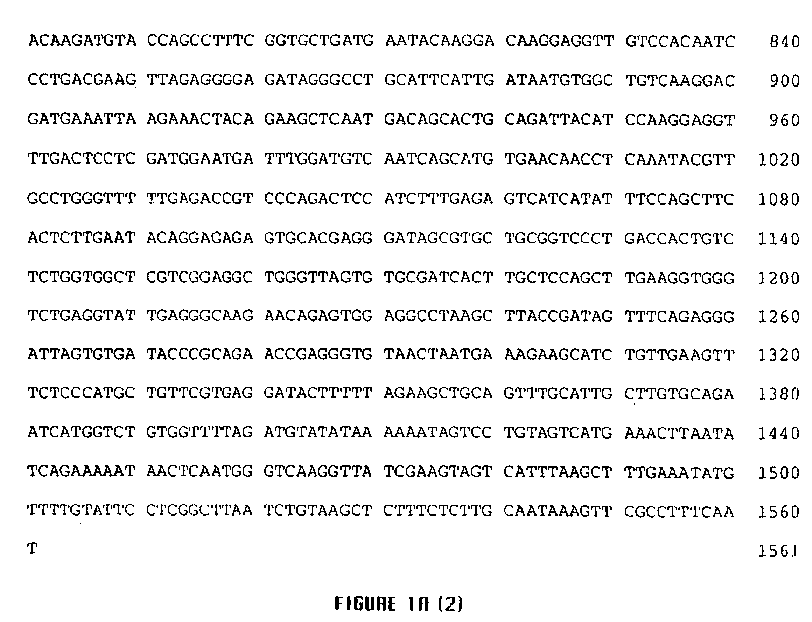

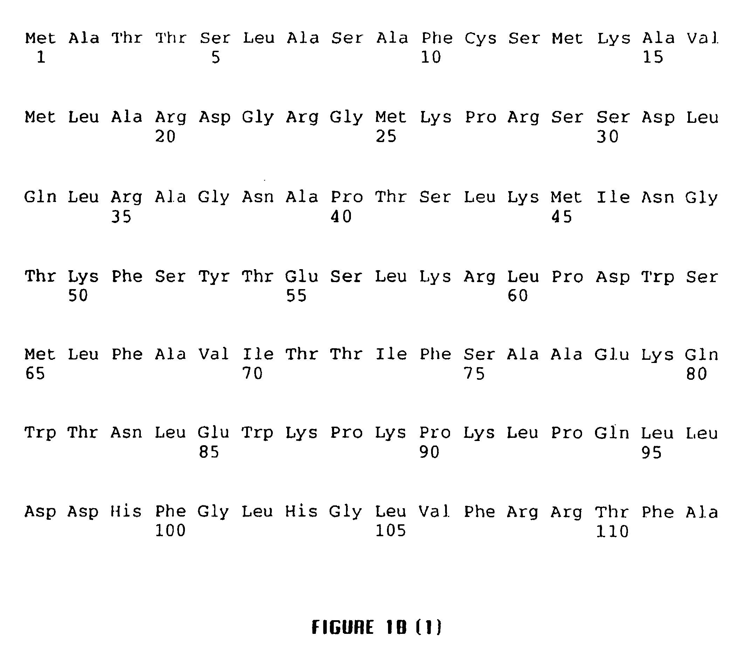

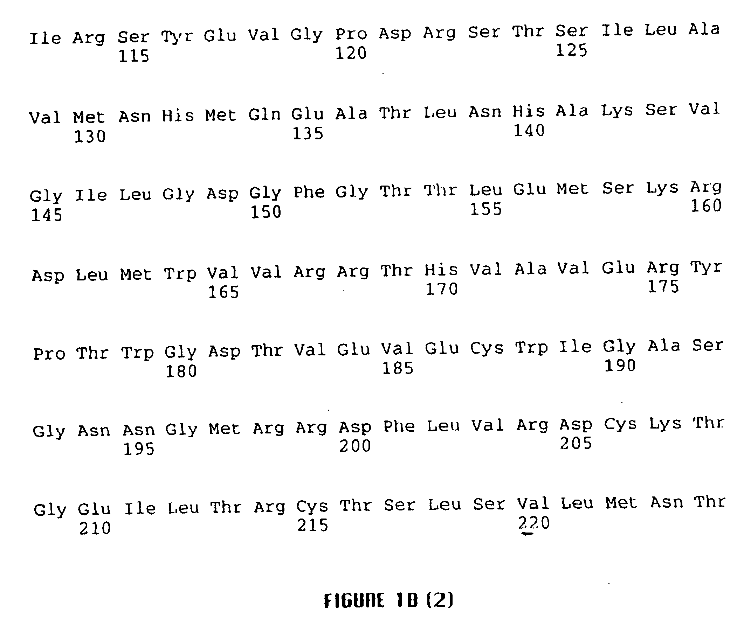

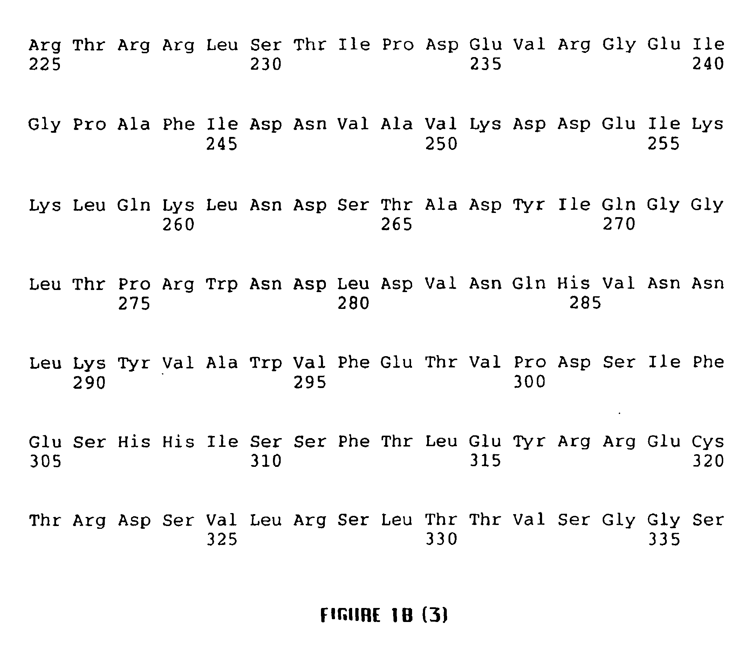

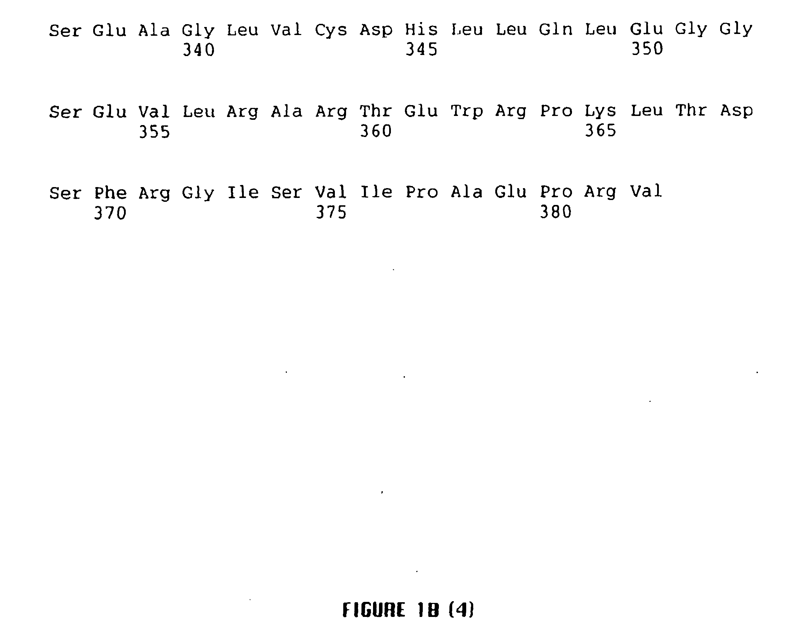

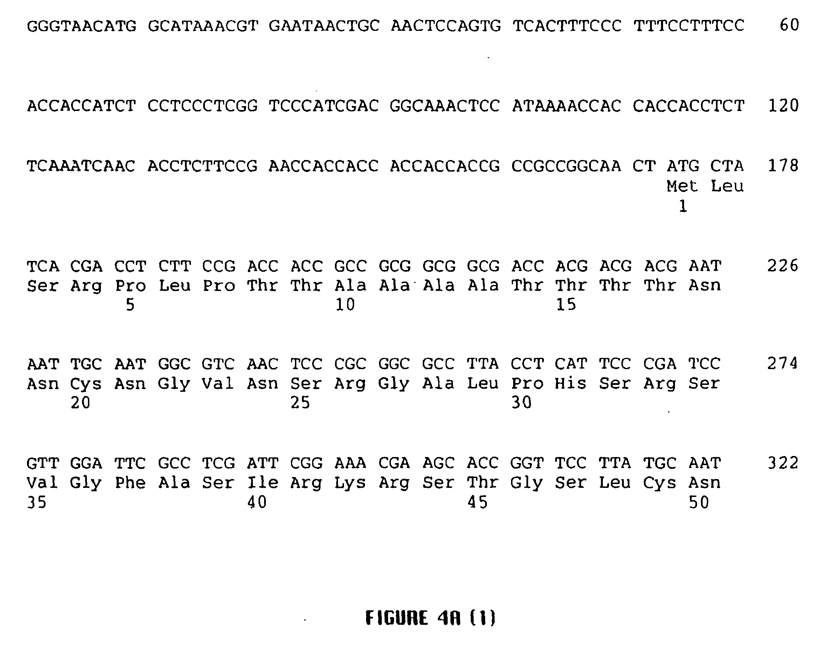

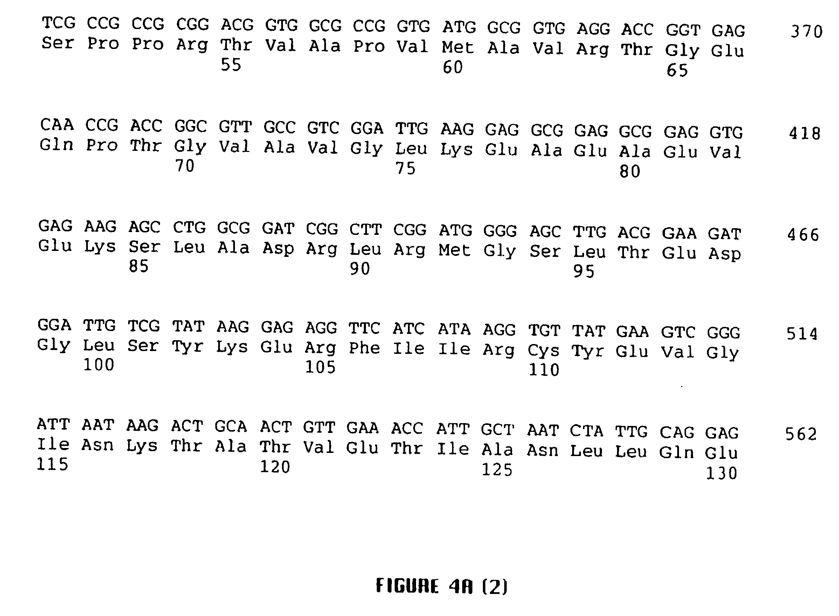

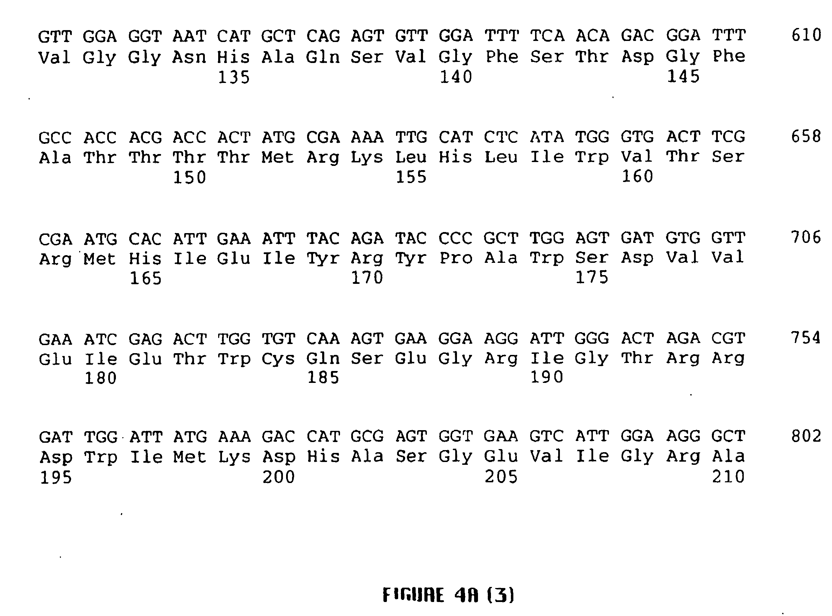

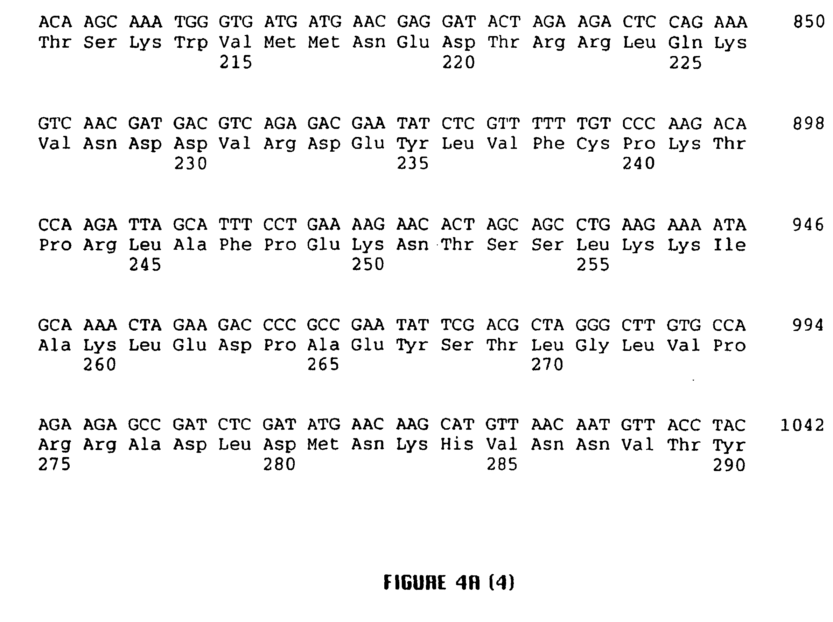

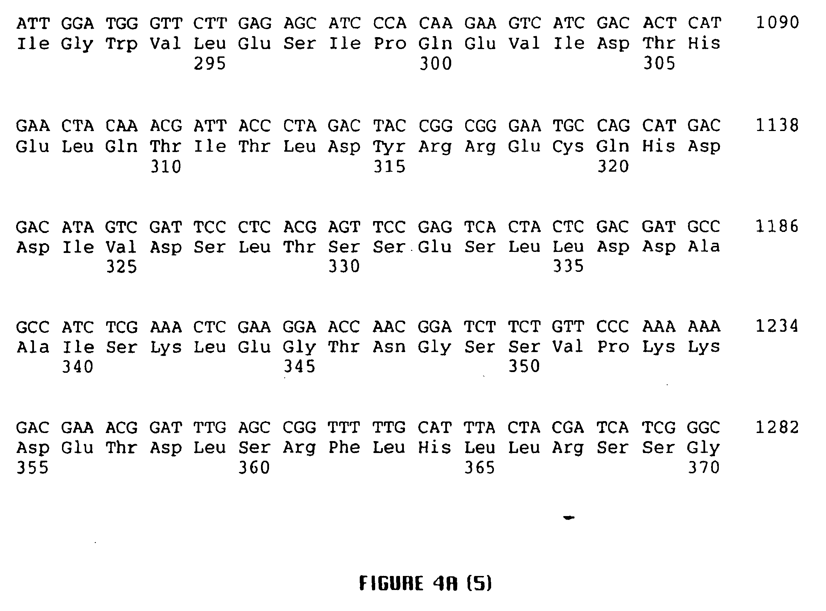

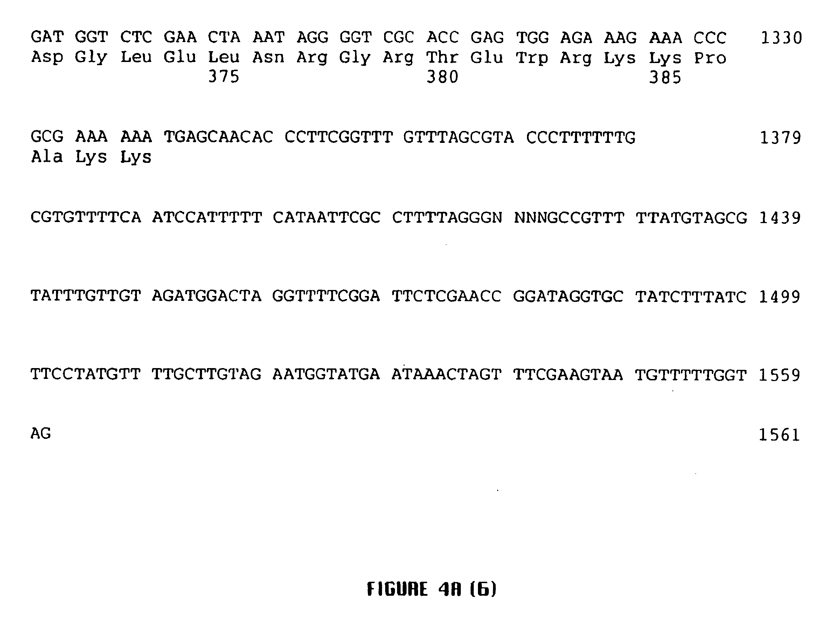

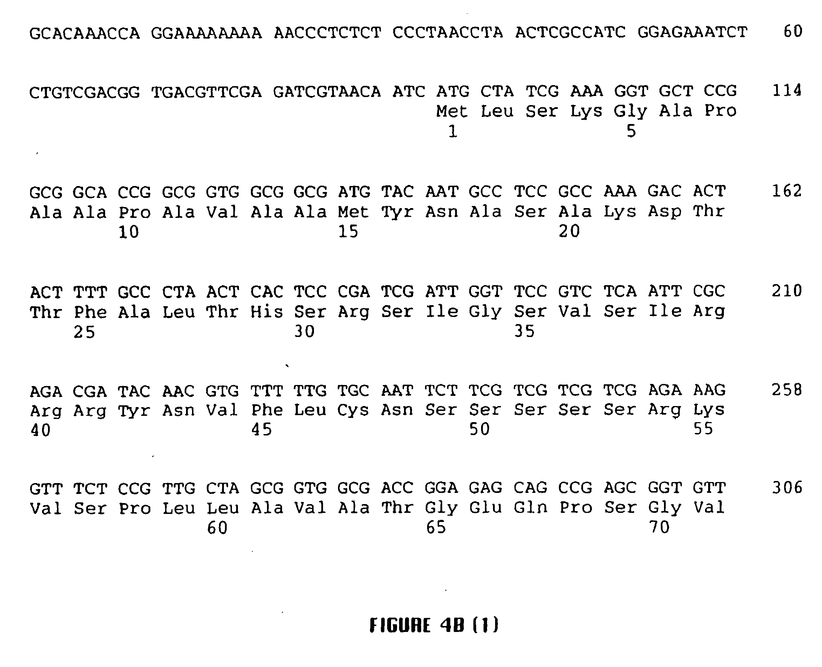

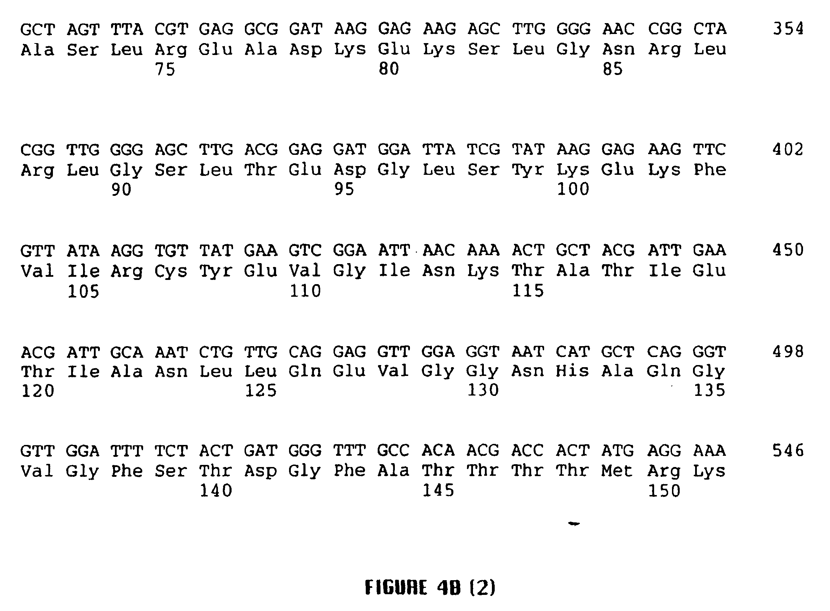

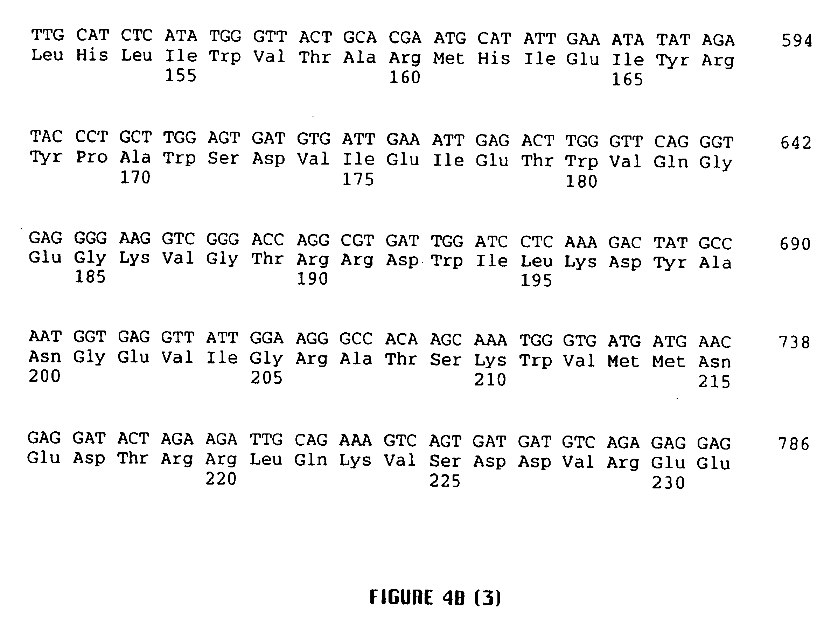

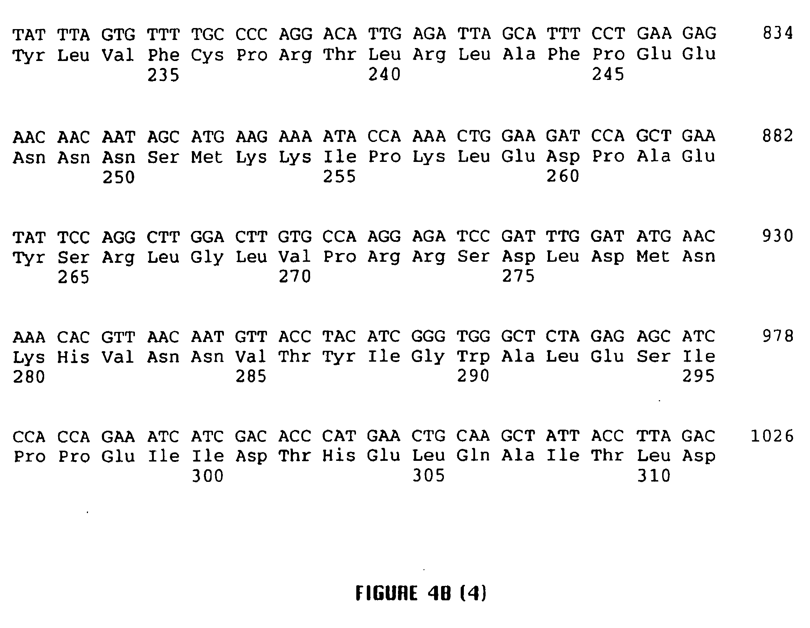

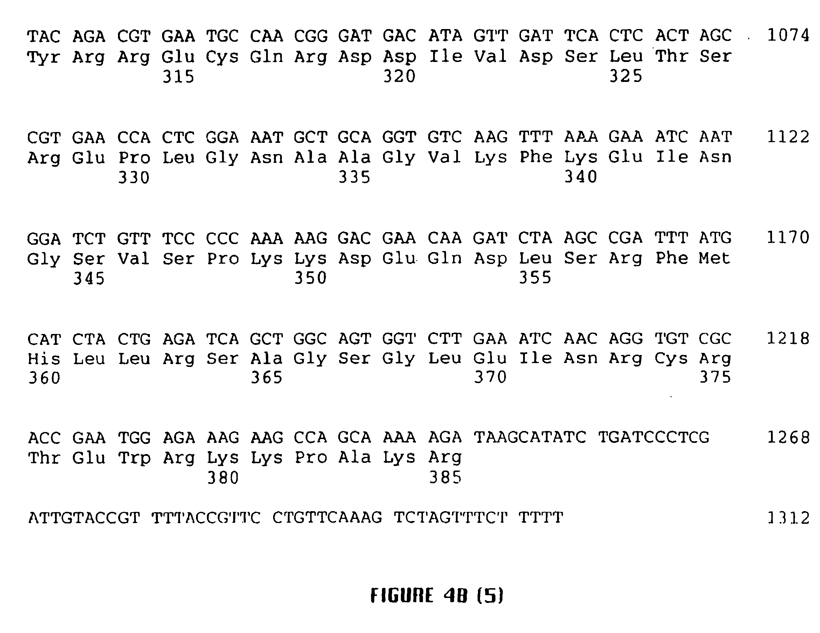

Additionally, means to increase the efficiency of translation may include the use of the complete structural coding sequence of the medium-chain thioesterase gene. Thus, use of the complete 5'-region of the bay thioesterase coding sequence, shown in Fig. 1B, may improve laurate production. Alternatively, if a medium-chain thioesterase has an unusual transit peptide sequence, i.e., one showing similarities with plastid thylakoid targeting, such as found with the bay thioesterase, then use of a more typical plant transit, such as found in safflower (Fig. 4), acyl carrier protein, or ssu may be substituted.

The present invention also provides the opportunuity for production of unsaturated fatty acids in a host cell, including plant cells. Plant medium-chain thioesterases, even from plants which do not have any unsaturated medium-chain fatty acids, may be active against such substrate. Hence, a plant medium-chain fatty acid may be used to provide unsaturated medium-chain fatty acids.

For example, expression of the bay thioesterase in E. coli results in the production of laurate (C12:0), myristate (C14:0) and also unsaturated species of medium-chain fatty acids (C12:1 and C14:1). The production of unsaturated fatty acids in *E. coli* is catalyzed by the action of β-hydroxydecanoyl thioester dehydrase. Sequence of the dehydrase is published (Cronan, *et al*., *J*. *Biol*. *Chem.* (1988)263:4641-4646) and thus can be inserted into a host cell of interest, including a plant cell, for use in conjunction with a medium-chain thioesterase.

When a plant medium-chain thioesterase is expressed in a bacterial cell, particularly in a bacterial cell which is not capable of efficiently degrading fatty acids, an abundance of medium-chain fatty acids can be produced and harvested from the cell. In some instances, medium-chain fatty acid salts form crystals which can be readily separated from the bacterial cells. Bacterial mutants which are deficient in acyl-CoA synthase, such as the E. *coli fad*D and *fad*E mutants, may be employed. In studies with *fad*D mutants, growth of *fad*D bay thioesterase transformants relative to the vector transformed control was severely retarded at 37°C, and less so at 25-30°C. Liquid cultures growing at the lower temperatures accumulated a precipitate and colonies formed on petri dishes at 25°C deposit large quantities of laurate crystals, especially at the surgace. These deposits, as identified by FAB-mass spectrometry were identified as laurate. After separation and quantitation by gas chromatography, it is estimated that the laurate crystals deposited by the *fad*D-bay thioesterase transformants on petri dises represented about 30-100% of the total dry weight of the producing bacteria.

When expression of the medium-chain thioesterase is desired in plant cells, various plants of interest include, but are not limited to, rapeseed (Canola and High Erucic Acid varieties), sunflower, safflower, cotton, *Cuphea*, soybean, peanut, coconut and oil palms, and corn. Depending on the method for introducing the recombinant constructs into the host cell, other DNA sequences may be required. Importantly, this invention is applicable to dicotyledyons and monocotyledons species alike and will be readily applicable to new and/or improved transformation and regulation techniques.

In any event, the method of transformation is not critical to the instant invention; various methods of plant transformation are currently available. As newer methods are available to transform crops, they may be directly applied hereunder. For example, many plant species naturally susceptible to *Agrobacterium* infection may be successfully transformed via tripartite or binary vector methods of *Agrobacterium* mediated transformation. In addition, techniques of microinjection, DNA particle bombardment, electroporation have been developed which allow for the transformation of various monocot and dicot plant species.

The following examples are provided by way of illustration and not by limitation.

### EXAMPLES

### Example 1 - Acyl-ACP Thioesterase cDNA Sequences

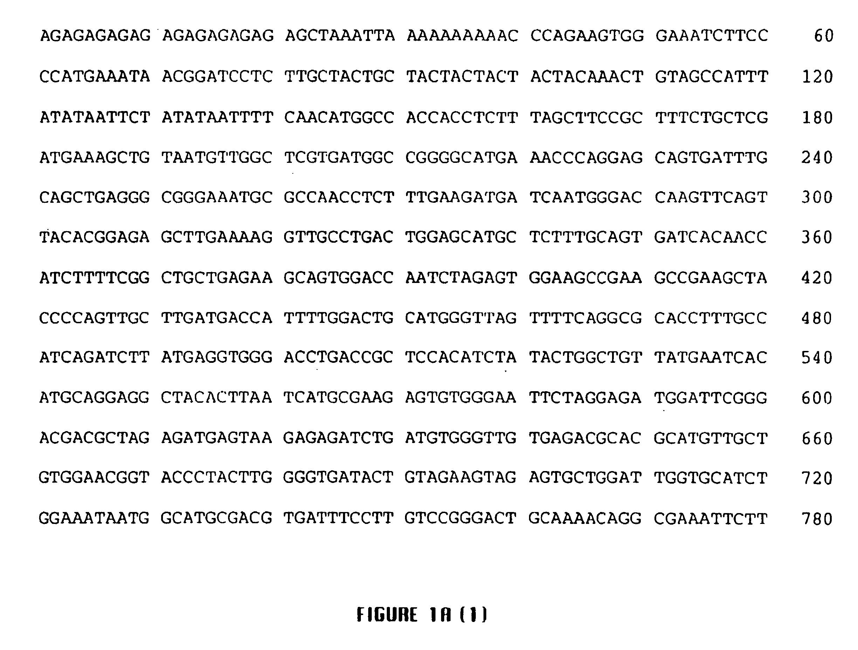

Sequence of a full length bay medium-chain thioesterase cDNA clone, pCGN3822, (3A-17), is presented in Fig. 1A.

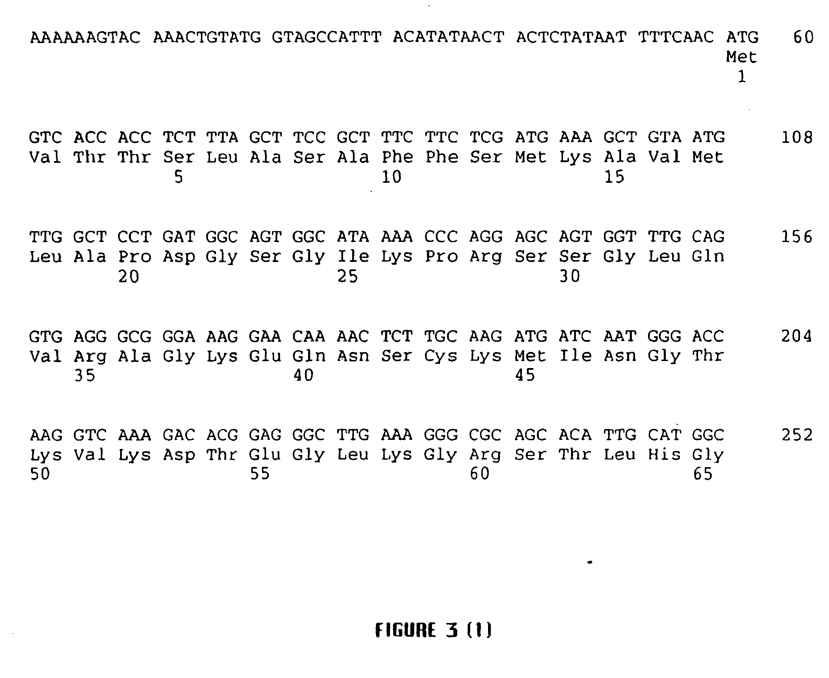

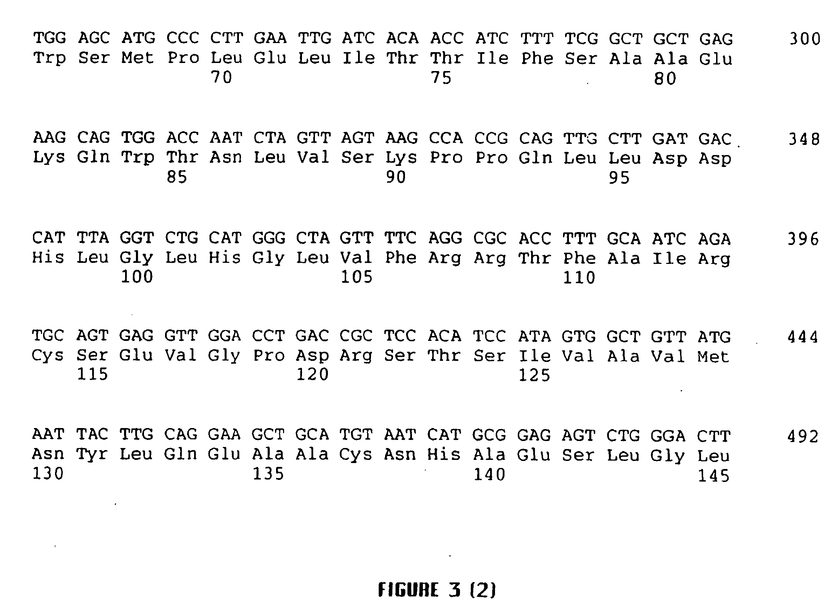

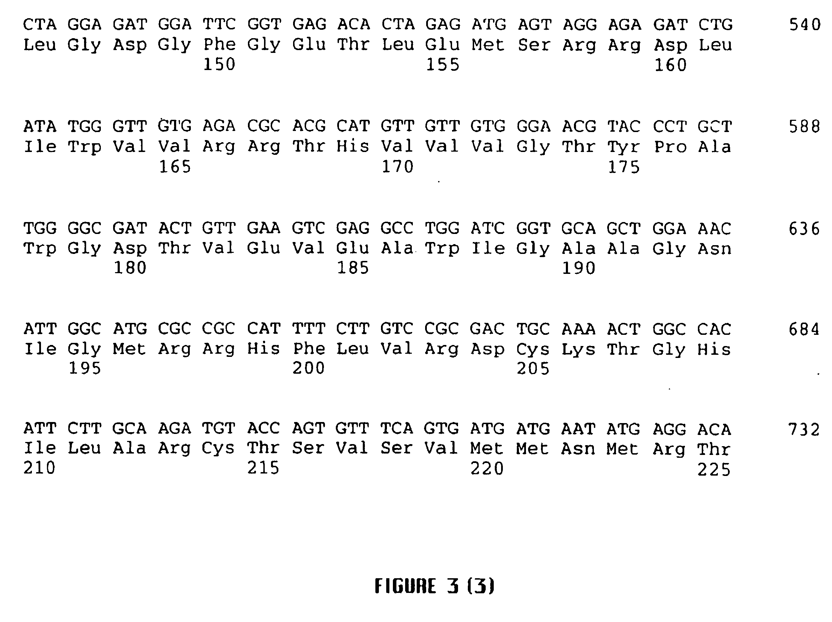

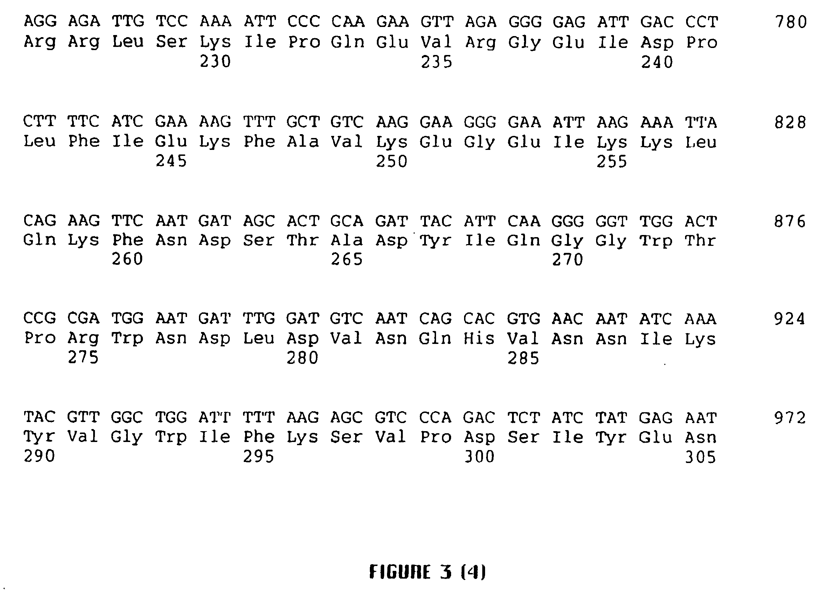

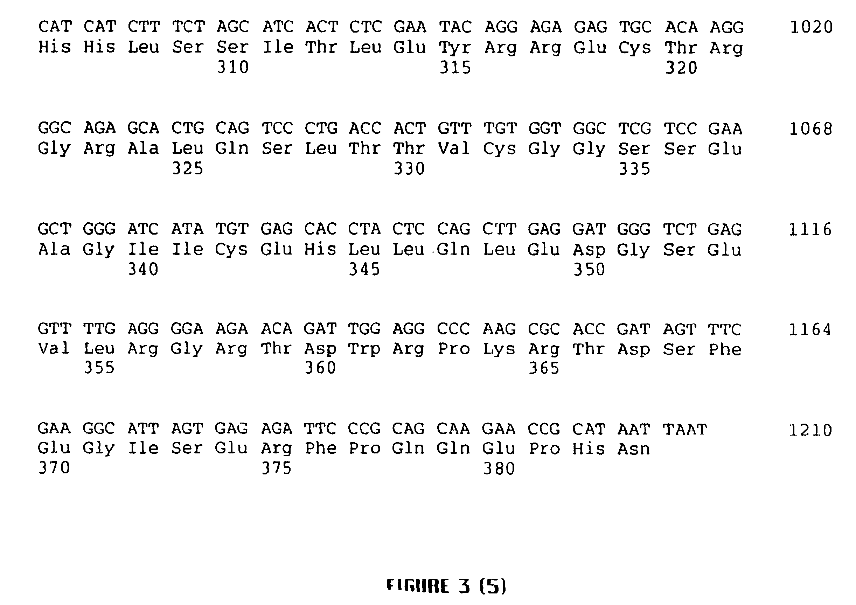

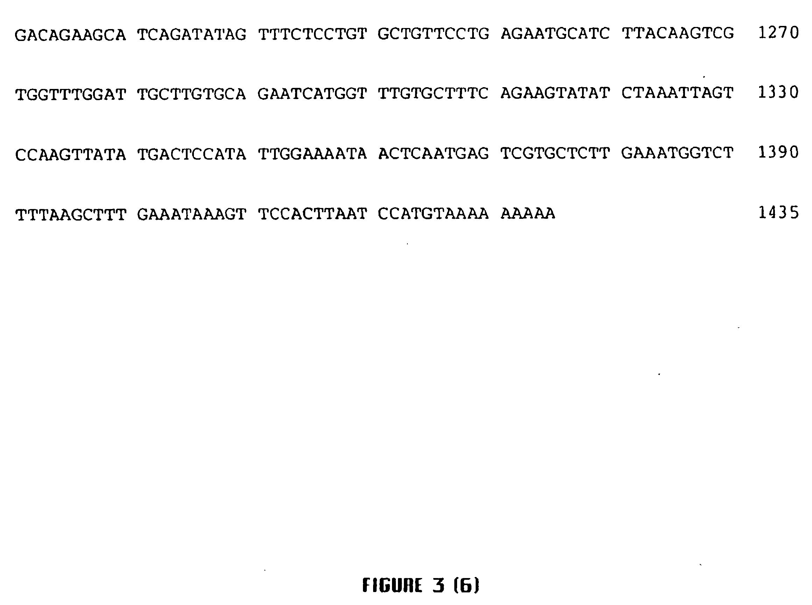

The translated amino acid sequence of the bay thioesterase beginning at the ATG codon at positions 145-147 is shown in Figure 1B. This ATG is surrounded by a sequence which matches the rules for plant initiation of translation and is therefore likely to be the initiation codon utilized *in vivo.* Using the ATG at bp 145 for initiation, a 382 amino acid polypeptide can be translated from the bay thioesterase mRNA. DNA sequence of second class of bay thioesterase genes is provided in Fig. 3.

The N-terminal sequence of the mature bay thioesterase, isolated from the developing seeds, starts at amino acid residue 84 of the derived protein sequence. The N-terminal 83 amino acids therefore represent sequence of a transit peptide. This sequence has features common to plastid transit peptides, which are usually between 40 and 100 amino acids long (Keegstra *et al*., *Ann. Rev. Plant Physiol. and Plant Mol. Biol.* (1989) *40*:471-501). A hydropathy plot of this transit peptide region reveals a hydrophobic domain at each end of the transit sequence. Other transit peptide sequences have been shown to contain similar hydrophobic N-terminal domains. The significance of this N-terminal domain is not known, but certain experiments suggest that lipid-mediated binding may be important for plastid import of some proteins (Friedman and Keegstra, *Plant Physiol.* (1989) *89*:993-999). As to the C-terminal domain, comparison of hydropathy plots of known imported chloroplastic stromal protein transit peptides (Keegstra *et al*, *supra)* indicates that these transit peptides do not have a hydrophobic domain at the C-terminus. However, preproteins destined to the thylakoid lumen of the chloroplast have an alanine-rich hydrophobic domain at the C-terminal end of their transit peptides (Smeekens *et al*., *TIBS* (1990) *15*:73-76). The existence of such a domain in the transit sequence of the bay thioesterase suggests that it has a double-domain transit peptide targeting this enzyme to the lumen of the thylakoid equivalent or to the intermembrane space. This is unexpected, since the substrate, acyl-ACP, has been detected in the stroma (Ohlrogge *et al*., *Proc. Nat. Acad. Sci.* (1979) *76*: 1194-1198). An alternative explanation for the existence of such a domain in the bay thioesterase preprotein is that it may represent a membrane anchor of the mature protein that is cleaved upon purification, leading to a sequence determination of an artificial N-terminus. The *in vivo* N-terminus of the mature thioesterase protein would then lie at a location further upstream than indicated by amino acid sequence analysis.

Gene bank searches with the derived amino acid sequence do not reveal significant matches with any entry, including the vertebrate medium-chain acyl-ACP thioesterase II (Naggert *et al*., *Biochem*. *J.* (1987) *243*:597-601). Also, the bay thioesterase does not contain a sequence resembling the fatty acid synthetase thioesterase active-site motif (Aitken, 1990 in *Identification of Protein Concensus Sequences, Active Site Motifs*, *Phosphorylation and other Post-translational Modifications* (Ellis Horwood, Chichester, West Sussex, England, pp. 40-147).

For comparison, isolation and sequence of a long-chain acyl-ACP thioesterase is provided. Sequence information from cyanogen bromide peptide sequences of safflower 34 and 40 kD thioesterase proteins is analyzed to obtain a peptide map of the safflower thioesterase. Homology comparisons of these peptides to the amino acid sequence of the bay thioesterase confirm the safflower thioesterase peptide map.

Degenerate oligonucleotide primers are designed from amino acid sequences of safflower thioesterase peptide sequences and used as primers in polymerase chain reactions (PCR) to obtain a fragment of a safflower thioesterase gene.

The thioesterase PCR gene product of the reaction is gel-purified and used as a probe to screen a safflower embryo cDNA library. Six clones are isolated; restriction mapping indicates that they fall into two gene classes. The nucleotide and translated amino acid sequences of a representative from each class, pCGN3264 (2-1) and pCGN3265 (5-2) are presented in Figure 4A and 4B. DNA sequence of pCGN3265 (5-2) with additional 3' untranslated sequence is shown in Figure 4C. Based on N-terminal amino acid sequence information, the amino terminal of the mature safflower thioesterases is assigned to the alanine residue at amino acid 61 of the translated amino acid sequences in Figure 4A and 4B.

Comparison of the deduced amino acid sequences of the two acyl-ACP thioesterase cDNA clones indicates that the mature proteins are 82% identical while the corresponding DNA sequences share 80% identity. Computer estimates of the isoelectric point of the two proteins differ considerably. The estimated pI for the mature protein encoded by 2-1 is 5.8, while that of the protein encoded by 5-2 is 8.1.

The results of safflower thioesterase purification indicated that there are potentially several forms of the safflower thioesterase. Two distinct molecular mass classes, as well as two separate peak fractions from chromatofocusing were observed. Both molecular mass species are represented in each activity peak. However, protein sequence analysis of each form indicates that these isoforms, are likely products of a single protein. The N-terminal sequence of each species is identical, and no differences in protein sequence of any of the internal CNBr fragments were observed. The different molecular weight species may be the result of a C-terminal peptide being removed either by processing *in vivo* or by degradation during the extraction and purification, perhaps during the acid precipitation step

While peptide sequence evidence indicates that all of the isoforms observed in purification of the safflower thioesterase may be derived from the same protein, two highly homologous but distinct classes of cDNAs were isolated from a safflower embryo cDNA library. Both classes encode an acyl-ACP thioesterase having preferential activity towards C18:1 substrates based on expression in E. coli. However, the peptide sequences data matches only the translated amino acid sequence from the 2-1 encoded protein (with allowance for minor discrepancies due to amino acid sequencing), and no peptides were found that uniquely correspond to the thioesterase encoded by the 5-2 gene. Possibly, the protein encoded by 5-2 is lower in abundance and is not a sufficiently prominent band to be considered for sequencing. Alternatively, the protein encoded by 5-2 may have been a minor component of the digested sample, with the result that the CNBr fragments were not sufficiently abundant to detect after SDS-PAGE and electroblotting. As examination of the predicted pI's of the two protein products indicates that 5-2 encodes a much more basic protein than does 2-1, the protein corresponding to 5-2 may have been eliminated during the acid precipitation step in purification.

### Example 2 - Expression of Acyl-ACP Thioesterases In E. coli

### Example 2A

Expression of bay thioesterase proteins in *E. coli* is described.

A truncated Bay (1200 bp) cDNA is expressed as a 30 kD protein in an *E. coli* host cell and data is provided demonstrating that the cDNA fragment confers upon the transformant an increased C12 acyl-ACP thioesterase activity.

A pET3a vector (Rosenberg, *et al*., *Gene* (1987) *56*:125-135) is used in an *E. coli* strain BL21 (PE3) (Studier and Moffat, *J*. *Mol*. *Biol*. (1986) *189*:113-130) host for this study. The pET3a vector contains a promoter and 33 bp of the 5' reading frame of bacteriophase T7. T7 polymerase is under the regulatory control of an isopropyl-b-D-thiogalactopyranoside (IPTG)-inducible lac UV5 promoter found in the *E. coli* BL21 (DE3) strain. Thus, by the addition of IPTG to *E*. *coli* BL21 (DE3) transformed with pET3a, the T7 promoter will be activated.

Constructs are prepared containing the truncated cDNA of Fig. 1 fused in reading frame by deletion of the *Bam*HI/*Eco*RI fragment and replacement of the thioesterase sequence. *E*. *coli* are transformed with pET3a constructs containing the thioesterase (pET3a-THI0) and unmodified pET3a as a control. The *E. coli* are grown at 37°C in liquid medium and expression is induced by the addition of 1mM IPTG. After 1 hour induction, cells are harvested by centrifugation, resuspended in assay buffer and lysed by sonication. Cell debris is removed by further centrifugation and the supernant used in activity assays as per Pollard *et al*., *Arch. Biochem & Biphys.* (1991) *281*:306-312.

**Table 1**

| *E*. *coli* Lysate | Assay Substrate | Hydrolysis Activity (mean cpm in ether extract) |
|---|---|---|
| pET3a | 8:0-ACP | 370 |
| " | 10:0-ACP | 787 |
| " | 12:0-ACP | 1028 |
| " | 14:0-ACP | 1271 |
| " | 16:0-ACP | 2848 |
| " | 18:1-ACP | 2877 |
| pET3a-THIO | 8:0-ACP | 349 |
| " | 10:0-ACP | 621 |
| " | 12:0-ACP | 2127 |
| " | 14:0-ACP | 1035 |
| " | 16:0-ACP | 1900 |
| " | 18:1-ACP | 2025 |

The results demonstrate that a lysate of control E. coli cells contains hydrolytic activity towards all the acyl-ACP substrates that were tested, with preference for the long-chain substrates. Comparing the pET3a-THI0 results with the control results it is evident that the pattern of substrate preferences differs. The transformant lysate shows greatly increased activity with 12:0-ACP in relation to the other substrates, as compared with the control lysate. This increased 12:0-ACP activity demonstrates that this cDNA fragment comprises sufficient of the the Bay 12:0-ACP thioesterase gene to produce active enzyme in *E. coli* cells.

In addition, the entire mature bay thioesterase protein is expressed as a lac fusion in *E. coli* cells. Sequence analysis of the full length bay thioesterase cDNA, pCGN3822, described in Example 1, reveals an *Xba*I site at base 394. Digestion at this *Xba*I site cleaves the coding region immediately 5' of the codon representing the leucine at amino acid position 72. This leucine has been identified as a candidate for the amino terminal residue as described in Example 1A.

An approximately 1200 bp fragment of pCGN3822 cDNA is generated by digestion with *Xba*I, which cuts at the postulated mature protein start site, as described above, and in the vector sequences flanking the 3' end of the cDNA. The *Xba*I fragment is cloned on XbaI digest of the minus version of a Bluescribe M13(+/-) (also called pBS+/-) cloning vector (Stratagene; San Diego, CA.). The thioesterase gene clone is inserted such that the mature protein is in reading frame with a portion of the lacZ gene of the Bluescribe vector and under control of the lac promoter.

The resulting construct, pCGN3823, and a control Bluescribe construct having the bay thioesterase gene inserted in the opposite orientation are transformed into *E. coli.* The *E. coli* cells are grown at 37°C in liquid medium and expression from the lac promoter is induced by addition of IPTG to a final concentration of 0.1mM IPTG. Following one hour of induction, cells are harvested, lysed and assayed as described above for the truncated bay thioesterase.

**Table 2**

| Induced *E*. *coli* Lysate | Dilution | Assay Substrate | Hydrolysis Activity (mean cpm in ether extract) |
|---|---|---|---|
| pCGN3823 | 1/4000 | 8:0-ACP | 0 |
| " | " | 10:0-ACP | 0 |
| " | " | 12:0-ACP | 1840 |
| " | " | 14:0-ACP | 116 |
| " | " | 16:0-ACP | 20 |
| " | " | 18:1-ACP | 5 |
| control | 1/4000 | 8:0-ACP | 0 |
| " | " | 10:0-ACP | 0 |
| " | " | 12:0-ACP | 0 |
| " | " | 14:0-ACP | 0 |
| " | " | 16:0-ACP | 13 |
| " | " | 18:1-ACP | 6 |

The results demonstrate that a lysate from *E. coli* cells expressing the postulated mature bay thioesterase enzyme has significantly greater activity towards a 12:0-ACP substrate than towards other ACP substrates of varying carbon chain length. In addition, this activity is more than two orders of magnitude greater than that in a lysate of *E. coli* cells expressing the truncated bay thioesterase. Studies are being conducted to determine if expression of the bay thioesterase protein in *E. coli* cells has an effect on the fatty acid composition of these cells. Initial studies failed to identify a substantial change in the fatty acid composites of the *E. coli* cells containing the bay thioesterase. However, analysis of larger samples of either pelleted transformed cells or the growth media from which the transformed cells have been pelleted, as described below, indicates a change in the fatty acid profile of the transformed cells. C12 fatty acids are produced in higher amounts in the cells containing the bay thioesterase as compared to untransformed control cells.

Approximately 100ml of *E. coli* control cells transformed with the plasmid vector Bluescribe (Stratagene; San Diego, CA) and cells transformed with the mature thioesterase construct are grown to an approximate O.D of 0.6 in ECLB (*E. coli* Luria broth) media, and pelleted by centrifugation. The cells and medium are extracted using an acidic method as follows. The pelleted cells are resuspended in 4ml of 5% (v/v) H₂SO₄ in methanol. The medium is recovered following centrifugation and 10ml of acetic acid is added. The sample is shaken vigorously with 50ml ether. The phases are allowed to separate and the lower layer is discarded. The ether layer is allowed to evaporate overnight resulting in 1-2ml of remaining solution. Four ml of 5% (v/v) H₂SO₄ in methanol is added to the remaining medium solution.

The following steps apply for fatty acid analysis of both the media solution and the pelleted cells described above. The cells or medium samples in H₂SO₄/methanol are transferred to screw-capped tubes and 2ml of toluene containing 0.5mg/ml of a C17 standard is added. The tubes are capped tightly, incubated at 90°C for 2 hours, after which 4ml of 0.9% (w/v) NaCl and 2ml of hexane are added. The samples are vortexed to mix thoroughly and then centrifuged for 5 minutes at 1500rpm. The upper (hexane) layer of each sample is then centrifuged for 5 minutes at 1000rpm in a table top centrifuge to separate any extracted fatty acid methyl esters that could be trapped within the layer of *E. coli* cells.

The samples are analyzed by gas-liquid chromatography (GC) using a temperature program to enhance the separation of components having 10 or fewer carbons. The temperature program used provides for a temperature of 140°C for 3 minutes, followed by a temperature increase of 5°C/minute until 230°C is reached, and 230°C is maintained for 11 minutes. Samples are analyzed on a Hewlett-Packard 5890 (Palo Alto, CA) gas chromatograph. Fatty acid content calculations are based on the internal C17 standard.

GC analysis indicates that approximately 70% of the fatty acids in the medium from the transformed cells are C12 fatty acids. This compares to levels of approximately 2% C12 fatty acids in the medium from the control cells. In addition, an approximately 2 fold increase in the C12 content of transformed cells over that of nontransformed cells is observed.

Substrate analysis of the bay thioesterase enzyme purified from developing seeds as described in Pollard, *et al*, *Supra,* is also conducted. Results are presented in Table 3 below.

**Table 3**

| Assay Substrate | Hydrolysis Activity (mean cpm in) Ether Extract |
|---|---|
| 8:0-ACP | 0 |
| 10:0-ACP | 0 |
| 12:0-ACP | 1261 |
| 14:0-ACP | 69 |
| 16:0-ACP | 12 |
| 18:1-ACP | 432 |

Comparison of the results of substrate analysis of the thioesterase in the *E*. *coli* extracts and as purified from developing bay seeds reveals that the activity profile of the enzyme from the two sources is essentially identical with respect to activity with C8, 10, 12, 14, and 16 ACP substrates. Although the enzyme purified from embryos is slightly more active with C18:1 substrates than is the *E*. *coli*-expressed thioesterase, this difference is believed due to activity of a long chain bay thioesterase which is not completely removed from the medium-chain thioesterase protein preparation.

### 1) Production of Laurate

For further studies, the bay thioesterase expression plasmid (pCGN3823) was established in an *E. coli* strain, *fad*D, which lacks the medium-chain specific acyl-CoA synthetase (Overath *et al*., *Eur. J. Biochem* (1969) 7:559-574) and is therefore unable to degrade laurate. Growth of *fad*D bay thioesterase transformants relative to the vector transformed control was studied at 25°, 30° and 37° C. In liquid culture bay thioesterase transformed *fad*D bacteria multiply, at all three temperatures, at nearly the same rate as the control during the exponential phase of growth. However, at 37°C, *fad*D cells harboring the bay thioesterase plasmid cannot be recovered from cultures nearing the stationary growth phase. In contrast the plasmids are stably contained at the lower temperatures for several days and these stationary cultures produce a significant amount of a precipitate which is soluble in methanol and ether.

Growth of fadD-bay thioesterase colonies on agar at is severely retarded 37°C , but only slightly so at the lower temperatures. The colonies formed on petri dishes at 25°C deposit large quantities of crystals, especially at the surface, but also in and at the surface of the cell free agar matrix. These crystal deposits were identified as potassium laurate by (FAB) mass spectrometry. After separation and quantitation by gas chromatography, the laurate crystals are estimated to represent up to 30% of the total dry weight of the producing bacteria.

### 2) Thiosterase Activity on Unsaturated Fatty Acyl Groups

In addition several new methyl ester peaks are present in the *fad*D-bay thioesterase, but not in the control *E. coli fad*D cells. Analyses indicate that two of these peaks represent 12:1 and 14:1 fatty acids. Thus, the bay thioesterase is able to hydrolyze fatty acyl-ACPs from both the saturated and unsaturated fatty acid synthetase pathways that are present in *E. coli.* The saturated pathway is intercepted essentially to 100% in late log phase, and the unsaturated pathway to about 70%. This causes a reduction of saturates in the phospholipids of the cells, substituted mainly by 16:1 and 18:1. The ratio of 12:1 to 14:1 accumulated is approximately 0.9 to 1, whereas the ratio of 12:0 to 14:0 accumulation is approximately 9 to 1. This may indicate that the chain-length specificity of the thioesterase on unsaturated fatty acyl ACPs is different from that on saturated substrates, or alternatively that the 14:1-ACP pool is much larger than the 12:1-ACP pool. In addition, the near complete interception of the saturated pathway appears to result in continuous synthesis of saturated fatty acids during the stationary phase of growth.

The striking difference in laurate accumulation levels between the *fad*D+ and the *fad*D transformants is in agreement with studies of bay thioesterase substrates specificity (Pollard, *et al*., *supra).* Laurate generated by the introduced bay thioesterase in *fad*D+ *E. coli* can be esterified to CoA, a much less effective substrate for the bay thioesterase, and subsequently degraded by β-oxidation or recycled for fatty acid synthesis. Therefore, only a small portion can accumulate and escape into the medium. In the *fad*D strain, laurate is not esterified to CoA and cannot by recycled. The observed slight growth retardation may indicate that the accumulation of laurate to such high levels results in a toxic effect on the *E. coli* host cells.

At 37°C, the synthesis of laurate in the *fad*D strain is tolerated only during exponential growth. The rapid loss of bay thioesterase plasmid containing cell titer at the end of the log phase may reflect a temperature dependence of laurate toxicity, or a physiological shift to stationary phase metabolism, which causes the introduced bay thioesterase activity to become lethal. The fatty acid composition of *E. coli* changes in aging cultures, and a reduced demand for saturated fatty acids at lower temperatures may lower the negative impact of the bay thioesterase expression at these temperatures. The pathway for unsaturated fatty acids in *E*. *coli* diverges at the C₁₀ stage and is most likely not intercepted by the bay thioesterase.

The accumulation of laurate in the medium is accompanied by deposition of smaller amounts of caprate (10:0). This is in contract with the the thioesterase activity profile where 14:0-ACP hydrolysis is more significant than 10:0-ACP hydrolysis. The high amount of bay thioesterase in these cells may effectively reduce the *in vivo* pool sizes of acyl-ACP's ≥ 12:0, so that less 14:0 acyl ACP substrate is available. The caprate production by the bay thioesterase in *E. coli* may indicate that this enzyme is responsible for both 10:0 and 12:0 fatty acid deposition in bay seeds.

### Example 2B

Expression of safflower thioesterase proteins in *E*. *coli* is described.

Safflower acyl-ACP thioesterase clones pCGN3264 and pCGN3265 are altered by site-directed mutagenesis to insert *Sal*I and *Nco*I sites immediately at the start of the mature protein coding region of these clones. The mature coding region plus 3'-untranslated sequences in the cDNA clones are removed as a *Nco*I/*Sma*I fragment and inserted into pET8c (Studier *et al*., 1990) that has been digested with *Bam*HI and treated with Klenow fragment of DNA polymerase to create a blunt end, and then cut with *Nco*I. The resulting expression constructs, pCGN3270 (2-1) and pCGN3271 (5-2) were designed to express the mature safflower acyl-ACP thioesterase cDNA sequences directly from the T7 promoter. For expression analysis, the constructs are transferred into *E. coli* strain BL21(DE3) containing the T7 RNA polymerase gene under control of the isopropyl β-D-thiogalactopyranoside (IPTG)-inducible *lac*UV5 promoter (Studier *et al*., *Methods Enzymol* (1990) *185*:60-89).

For thioesterase activity assay, cells containing pCGN3270, pCGN3271, or pET8c as a control are grown at 37°C to an OD₆₀₀ of ∼0.5 in 2YT (16 g tryptone, 10 g yeast extract, 5 g NaCl per liter, pH 7.0) containing 0.4% glucose and 300 µg/ml penicillin. Induction is achieved by the addition of IPTG to 0.4 mM and 1.5 hours further growth. Ten-ml aliquots of culture are harvested by centrifugation and the pelleted cells stored at -70°C. Prior to assay, pellets are resuspended in 500 µl of thioesterase assay buffer and sonicated for three bursts of 20 seconds each. Protein concentrations are determined using the Bio-Rad Protein Assay.

Total protein profiles of *E. coli* containing pCGN3270 and pCGN3271 are analyzed by SDS-PAGE. In each case a new protein band is observed in the IPTG-induced cultures relative to the pET8c control. Although the computer-predicted molecular weight of the 2-1 and 5-2 encoded proteins are very similar, the mobility of these proteins as expressed from pCGN3270 and pCGN3271 is significantly different. The protein encoded by pCGN3270 has a mobility of approximately 40 kD, while the protein encoded by pCGN3271 is approximately 36 kD. The induced proteins were subjected to N-terminal sequencing to confirm their identity. In each case, the protein sequence matched that predicted by the cDNA. In addition, the nucleotide sequence of the 3' region of the 5-2 cDNA insert in pCGN3271 was resequenced to ensure that no premature stop codons had been introduced during the cloning steps.

Total extracts of cells expressing either pET8c (control), pCGN3270, or pCGN3271 are assayed for thioesterase activity using 18:1-ACP. The 18:1-ACP thioesterase activity in cells containing pCGN3270 and pCGN3271 is ∼100- and 50-fold higher respectively, than the activity in control cells. To further characterize the safflower acyl-ACP thioesterase, the chain-length specificity of the thioesterase activities expressed from the cDNA clones is tested for a variety of acyl-ACP substrates, and compared to control thioesterase activities of *E. coli* and a crude safflower embryo extract. The pCGN3270 and pCGN3271 cultures contain thioesterase activity characteristic of safflower embryos, i.e. much higher preference for 18:1-ACP vs. 18:0-ACP as compared to control *E. coli.* Between the two safflower thioesterase clones, the activity expressed from pCGN3271 displays a slightly broader specificity for the saturated 18:0-ACP and 16:0-ACP substrates.

### Example 3 - Constructs & Methods for Plant Transformation

A. Constructs for expression of bay thioesterase in plant cells which utilize phaseolin, napin, CaMV35S and Bce4 promoter regions are prepared as follows.

### Phaseolin/thioesterase

A 1.45kb fragment of pCGN3822 (3A-17) is obtained by digestion with *Bal*I and *Sal*I. The *Bal*I site is located at position 149 of the cDNA insert, and the *Sal*I site is in the polylinker located 3' to the cDNA insert. Thus, this fragment contains the entire thioesterase coding region and the entire cDNA 3' region, including the polyadenylation signal, AAATAA, located at bases 1447-1452, and also contains the restriction digestion sites *Kpn*I, *Sma*I, *Xba*I and *Sal*I located directly 3' to the cDNA.

An 850bp *Bgl*II fragment of the β-phaseolin 5' noncoding region was obtained from p8.8pro (Hoffman *et al*. (1987) *EMBO J.* 6:3213-3221) and cloned into pUC9 (Vieira and Messing, *supra*) at the *Bam*HI site to yield pTV796. The phaseolin fragment in pTV796 is oriented such that *Sma*I site of pUC9 is located 3' to the phaseolin promoter. An ∼850bp fragment is generated by digestion of pTV796 with *Hin*dIII and *Sma*I and gel-purified.

The phaseolin promoter (*Hin*dIII/*Sma*I) and thioesterase coding region (*Bal*I/*Sal*I) are joined by three way ligation into a Bluescript (Stratagene) cloning vector that has been digested with *Hin*dIII and *Sal*I. The resulting plasmid contains the phaseolin promoter/thioesterase construct on a *Hin*dIII/*Sal*I fragment that is flanked by various restriction sites, including a 5' *Bam*HI site and a 3' *Kpn*I site. No additional plant 3' noncoding region is provided as the thioesterase fragment contains a polyadenylation signal. The phaseolin promoter/thioesterase fragment may be obtained by digestion with *Bam*HI and *Kpn*I, or alternatively by partial digestion with *Xba*I, and ligated into an appropriate binary vector, such as pCGN1557 or pCGN1578 (McBride and Summerfelt, (1990) *Plant Mol*. *Biol*. *14*:269-276), for plant transformation. Ligation of the phaseolin promoter/thioesterase fragment, resulting from *Bam*HI and *Kpn*I digestion, into pCGN1578 results in pCGN3821.

### 35S/thioesterase/mas

An *Bal*I/*Pst*I fragment of the thioesterase cDNA 3A-17 containing approximately 1200bp, and including the entire coding region, is obtained by partial digestion with restriction enzymes *Bal*I and *Pst*I and gel-purifications of the 1200bp fragment. The fragment is ligated into a plasmid cloning vector, such as a Bluescript vector (Stratagene Cloning Systems; La Jolla, CA), that has been digested with *Pst*I and *Bam*HI, and the *Bam*HI site filled in using the Klenow fragment of DNA Polymerase I. In this procedure, the *Bam*HI site is restored by ligation to the *Bal*1 site of the thioesterase cDNA.

The resulting plasmid is partially digested with *Bam*HI and *Eco*RI to obtain the approximately 1200bp thioesterase fragment. This fragment is then cloned into an approximately 4.4kb *Bam*HI/*Eco*RI DNA fragment which contains approximately 0.94kb of 5' noncoding sequence from a cauliflower mosaic (CaMV) 35S gene (immediately 5' to the *Bam*HI site), approximately 0.77kb of 3' noncoding sequence from an *Agrobacterium tumefaciens* manopine synthase (mas) gene (immediately 3' to the *Eco*RI site), and a pUC19 (New England BioLabs, Beverly, MA) backbone. The *Bam*HI/*Eco*RI DNA fragment is obtained by partial digestion of a larger plasmid vector and gel purification of the desired 4.4kb fragment. The 35S 5' region is from bases 6492 to 7433 of strain CM1841 (Gardner, *et al*. (1981) *Nucl*. *Acids Res*. *9*:2871-2887), which is from about -640 to about +2 in relation to the transcription start site. The mas 3' noncoding region is from about bases 19,239 to 18,474 of octopine Ti plasmid pTiA6 (numbering corresponds to that of closely related pTi15955 as reported by Barker *et al*. (*Plant Mol*. *Biol*. (1983) *2*:335-350)).

The resulting 35S/thioesterase/mas plasmid is digested at flanking *Bgl*II sites and cloned into a *Bam*HI digested binary vector, such as pCGN1557 or pCGN1578 (McBride and Summerfelt, *supra*).

### Bce4/thioesterase

A 1.45kb thioesterase cDNA *Bal*I/*Sal*I fragment is prepared as described above. A Bce4 expression cassette, pCGN1870, which provides for preferential expression in early seed development is described in copending US Patent Application Serial No. 07/494,722.

An approximately 1kb fragment of the Bce4 5' noncoding region whose 3' end is immediately 5' to the Bce4 start codon, is obtained by digestion of pCGN1870 with XbaI and *Xho*I and gel purification of the resulting 1kb fragment.

The Bce4 promoter (*Xba*I/*Xho*I) and thioesterase coding region (*Bal*I/*Sal*I) are joined by three way ligation into a Bluescribe (Stratagene) cloning vector that has been digested with *Xba*I and *Sal*I. The resulting plasmid contains the Bce4 promoter/thioesterase construct on a *Xba*I/*Sal*I fragment that is flanked by various restriction sites, including a 5' *Bam*HI site and a 3' *Kpn*I site. No additional plant 3' noncoding region is provided as the thioesterase fragment contains a polyadenylation signal. The Bce4 promoter/thioesterase fragment may be obtained by digestion with *Bam*HI and partial digestion with *Kpn*I (or *Asp*718 which has the same recognition sequence), or alternatively by partial digestion with *Xba*I, and ligated into an appropriate binary vector, such as pCGN1557 or pCGN1578 (McBride and Summerfelt, supra), for plant transformation. Ligation of the Bce4 promoter/thioesterase fragment, resulting from *Bam*HI and *Kpn*I digestion, into pCGN1578 results in pCGN3820.

### Napin/thioesterase/napin

The napin expression cassette, pCGN1808, is described in copending US Patent Application serial number 07/550,804. pCGN1808 is modified to contain flanking restriction sites to allow movement of only the expression sequences and not the antibiotic resistance marker to binary vectors such as pCGN1557 (McBride and Summerfelt, *supra*). Synthetic oligonucleotides containing *Kpn*I, *Not*I and *Hin*dIII restriction sites are annealed and ligated at the unique *Hin*dIII site of pCGN1808, such that only one *Hin*dIII site is recovered. The resulting plasmid, pCGN3200 contains unique *Hin*dIII, *Not*I and *Kpn*I restriction sites at the 3'-end of the napin 3'-regulatory sequences as confirmed by sequence analysis.

The majority of the napin expression cassette is subcloned from pCGN3200 by digestion with *Hin*dIII and *Sac*I and ligation to *Hin*dIII and *Sac*I digested pIC19R (Marsh, *et al*. (1984) *Gene 32*:481-485) to make pCGN3212. The extreme 5'-sequences of the napin promoter region are reconstructed by PCR using pCGN3200 as a template and two primers flanking the SacI site and the junction of the napin 5'-promoter and the pUC backbone of pCGN3200 from the pCGN1808 construct. The forward primer contains *Cla*I, *Hin*dIII, *Not*I, and *Kpn*I restriction sites as well as nucleotides 408-423 of the napin 5'-sequence (from the EcoRV site) and the reverse primer contains the complement to napin sequences 718-739 which include the unique *Sac*I site in the 5'-promoter. The PCR was performed using in a Perkin Elmer/Cetus thermocycler according to manufacturer's specifications. The PCR fragment is subcloned as a blunt-ended fragment into pUC8 (Vieira and Messing (1982) *Gene 19*:259-268) digested with *Hin*cII to give pCGN3217. Sequenced of pCGN3217 across the napin insert verifies that no improper nucleotides were introduced by PCR. The napin 5-sequences in pCGN3217 are ligated to the remainder of the napin expression cassette by digestion with *Cla*I and *Sac*I and ligation to pCGN3212 digested with *Cla*I and *Sac*I. The resulting expression cassette pCGN3221, is digested with HindIII and the napin expression sequences are gel purified away and ligated to pIC20H (Marsh, *supra*) digested with *Hin*dIII. The final expression cassette is pCGN3223, which contains in an ampicillin resistant background, essentially identical 1.725 napin 5' and 1.265 3' regulatory sequences as found in pCGN1808. The regulatory regions are flanked with *Hin*dIII, *Not*I and *KpnI* restriction sites and unique *Sal*I, *Bgl*II, *Pst*I, and *Xho*I cloning sites are located between the 5' and 3' noncoding regions.

The 1200bp *Bal*I/*Pst*I thioesterase cDNA fragment described above is cloned into the napin expression cassette, pCGN3223, which has been digested with *Sal*I, and the *Sal*I site filled in using the Klenow fragment of DNA Polymerase I, followed by digestion with *Pst*I. The *Sal*I site is reconstituted in this ligation.

The napin/thioesterase/napin plasmid generated by these manipulations is digested with *Bam*HI and partially digested with *Kpn*I to generate an approximately 3.3kb fragment. This fragment contains ∼1.7kb of napin 5' noncoding sequence, the ∼1200bp *Bal*I/*Pst*I thioesterase cDNA fragment and ∼0.33kb of 3' napin noncoding region, the rest of the 1.265kb of the napin 3' having been deleted due to the *Bam*HI site in this region. The ∼3.3kb fragment is ligated to *Kpn*I/*Bam*HI digested pCGN1557 or pCGN1578 (McBride and Summerfelt, *supra)* for plant transformation. Insertion of the ∼3.3kb fragment into pCGN1578 results in pCGN3816.

### Napin/thioesterase

An approximately 1.5kb fragment of the full length thioesterase cDNA is obtained by partial digestion of pCGN3822 with *Bam*HI and *Kpn*I and subsequent gel-purification of the resulting 1.5kb fragment. The *Bam*HI site is at nucleotide 74 of the cDNA sequence and the *Kpn*I site is in the vector polylinker located 3' to the cDNA insert. Thus, this fragment contains the entire thioesterase coding region, including the ATG codon at positions 145-147, and the entire cDNA 3' region, which contains a polyadenylation signal as described above.

An approximately 1.7kb fragment of the napin 5' noncoding region is obtained by digestion of pCGN3223 (described above) with *Hin*dIII and *Bgl*II and subsequent gel-purification of the 1.7 kb fragment.

The napin promoter (*Hin*dIII/*Bgl*II) and the thioesterase coding region (*Bam*HI/*Kpn*I) are joined by a three fragment ligation into a binary vector, such as pCGN1557 or pCGN1578 (McBride and Summerfelt, *supra*) that is digested with *Hin*dIII and *Kpn*I. In this reaction, the complementary overhanging ends of the *Bam*HI and *Bgl*II sites allows fusion of the 3' end of the napin fragment to the 5' end of the thioesterase fragment. The resulting plasmid for plant transformation from ligation into pCGN1578, pCGN3824, contains the thioesterase cDNA positioned for expression under the regulatory control of the napin promoter. No additional plant 3' noncoding region is provided as the thioesterase fragment contains a polyadenylation signal.

### Napin/thioesterase/napin

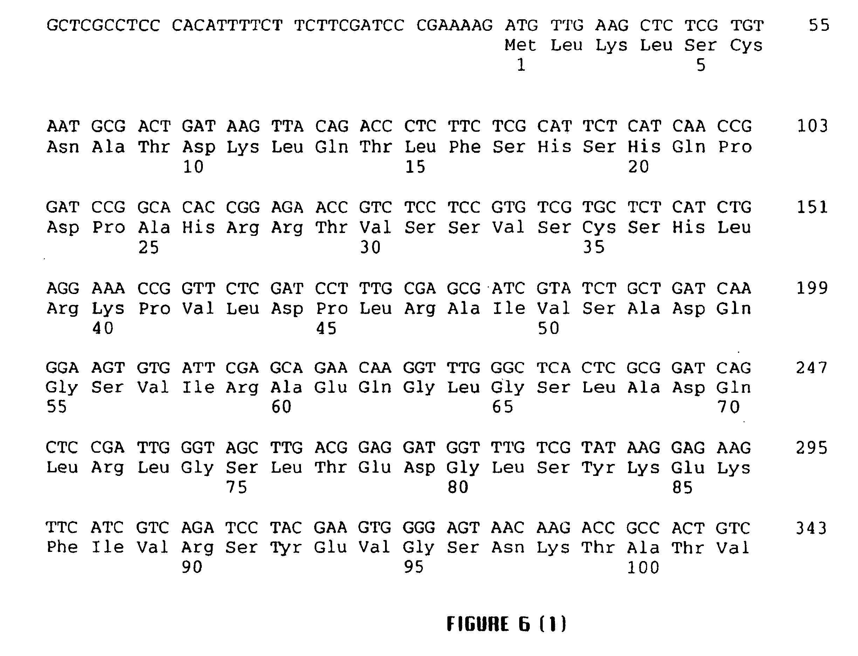

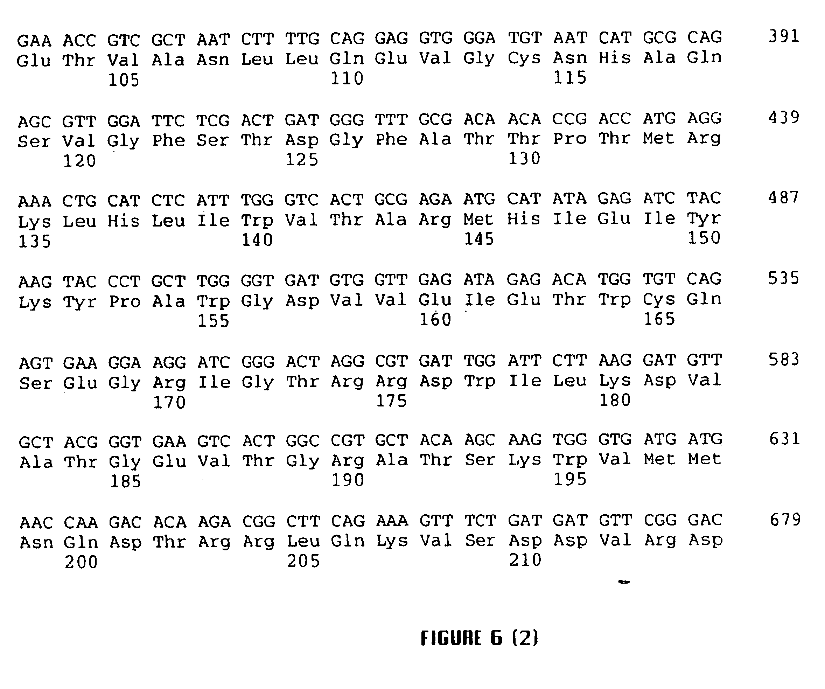

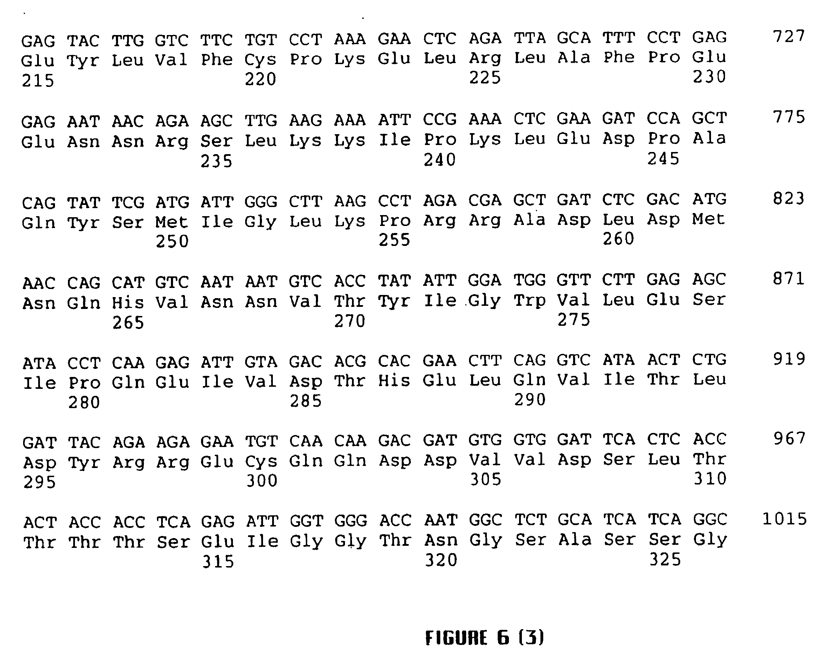

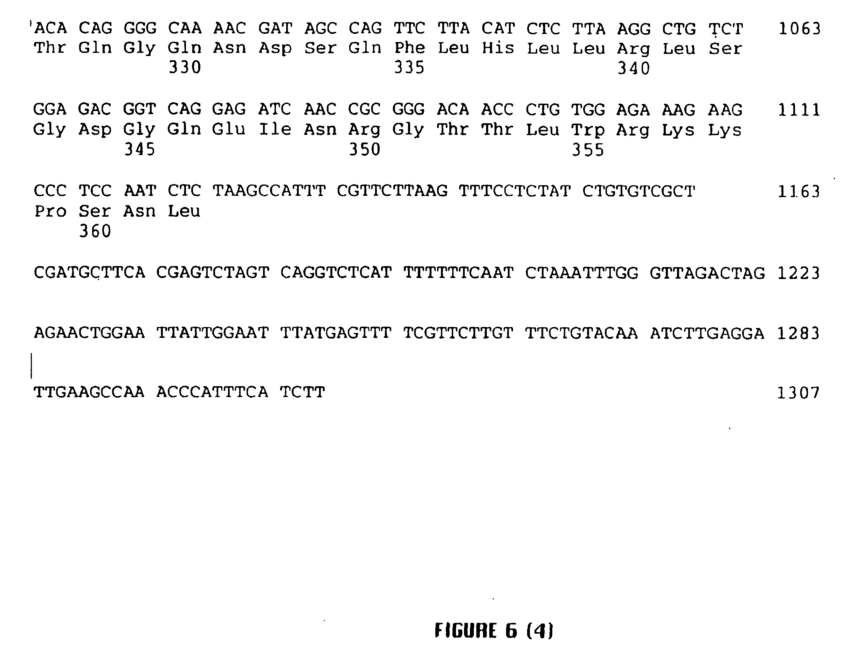

A construct for expression of thioesterase under the transcriptional and translational control of napin promoter and 3' transcriptional termination regions is made as follows. pCGN3822 (described above) is engineered using PCR techniques to insert a *Bam*HI site immediately 5' to the thymine nucleotide at position 140 (5 bases upstream of the ATG start codon) of the bay thioesterase sequence shown in Figure 6A (SEQ ID NO:41), resulting in pCGN3826. An approximately 1225bp fragment containing the entire thioesterase encoding region is obtained from pCGN3826 as a *Bam*HI to *Pst*I fragment and ligated into *Bgl*II/*Pst*I digested pCGN3223, the napin expression cassette described above, resulting in pCGN3827. A vector for plant transformation, pCGN3828, is constructed by partially digesting pCGN3827 with *Kpn*I and *Bam*HI, and cloning the approximately 3.2kb fragment containing the napin 5'/ thioesterase/ napin 3' construct into *Kpn*I/*Bam*HI digested pCGN1578 (McBride and Summerfelt, *supra*).

A construct, pCGN3837, is prepared which is similar to pCGN3828, but has the bay transit peptide coding region replaced with a sequence encoding the safflower thioesterase transit peptide and 6 amino acids of the mature safflower thioesterase from clone 2-1. The safflower fragment for this construct may be prepared using PCR techniques to provide convenient restriction digestion sites. Another construct having napin 5' and 3' regulatory regions is prepared which replaces the region encoding the bay thioesterase transit peptide and the first 11 amino acids of the mature bay thioesterase protein with a sequence encoding the safflower thioesterase transit peptide and the first 31 amino acids of the mature safflower thioesterase protein.

An appropriate *Agrobacterium* strain is transformed with the binary constructs and used to generate transformed laurate producing plants. Seeds are collected and analyzed as described above to determine efficiency of plastid transport and oil composition.

B. A variety of methods have been developed to insert a DNA sequence of interest into the genome of a plant host to obtain the transcription or transcription and translation of the sequence to effect phenotypic changes.

### Brassica Transformation

Seeds of *Brassica napus* cv. Westar are soaked in 95% ethanol for 2 min. surface sterilized in a 1.0% solution of sodium hypochlorite containing a drop of Tween 20 for 45 min., and rinsed three times in sterile, distilled water. Seeds are then plated in Magenta boxes with 1/10th concentration of Murashige minimal organics medium (Gibco; Grand Island,NY) supplemented with pyriodoxine (50µg/l), nicotinic acid (50µg/l), glycine (200µg/l), and 0.6% Phytagar (Gibco) pH 5.8. Seeds are germinated in a Percival chamber at 22°C. in a 16 h photoperiod with cool fluorescent and red light of intensity approximately 65µ Einsteins per square meter per second (µEm⁻²S⁻¹).

Hypocotyls are excised from 5-7 day old seedlings, cut into pieces approximately 4mm in length, and plated on feeder plates (Horsch *et al*., *Science* (1985) *227*:1229-1231). Feeder plates are prepared one day before use by plating 1.0ml of a tobacco suspension culture onto a petri plate (100x25mm) containing about 30ml MS salt base (Carolina Biological, Burlington, NC) 100mg/l inositol, 1.3mg/l thiamine-HCl, 200mg KH₂PO₄ with 3% sucrose, 2,4-D (1.0mg/l), 0.6% w/v Phytagar, and pH adjusted to 5.8 prior to autoclaving (MS 0/1/0 medium). A sterile filter paper disc (Whatman 3mm) is placed on top of the feeder layer prior to use. Tobacco suspension cultures are subcultured weekly by transfer of 10ml of culture into 100ml fresh MS medium as described for the feeder plates with 2,4-D (0.2mg/l), Kinetin (0.1mg/l). In experiments where feeder cells are not used hypocotyl explants are cut and placed onto a filter paper disc on top of MS0/1/0 medium. All hypocotyl explants are preincubated on feeder plates for 24 h. at 22°C in continuous light of intensity 30µEm⁻²S⁻¹ to 65µEM⁻²S⁻¹.

Single colonies of *A*. *tumefaciens* strain EHA 101 containing a binary plasmid are transferred to 5ml MG/L broth and grown overnight at 30°C. Hypocotyl explants are immersed in 7-12ml MG/L broth with bacteria diluted to 1x10⁸ bacteria/ml and after 10-25 min. are placed onto feeder plates. Per liter MG/L broth contains 5g mannitol, 1g L-Glutamic acid or 1.15g sodium glutamate, 0.25g kH₂PO₄, 0.10g NaCl, 0.10g MGSO₄·7H₂O, 1mg biotin, 5g tryptone, and 2.5g yeast extract, and the broth is adjusted o pH 7.0. After 48 hours of co-incubation with *Agrobacterium,* the hypocotyl explants are transferred to B5 0/1/0 callus induction medium which contains filter sterilized carbenicillin (500mg/l, added after autoclaving) and kanamycin sulfate (Boehringer Mannheim; Indianapolis, IN) at concentrations of 25mg/l.

After 3-7 days in culture at 65µEM⁻²S⁻¹ continuous light, callus tissue is visible on the cut surface and the hypocotyl explants are transferred to shoot induction medium, B5BZ (B5 salts and vitamins supplemented with 3mg/l benzylaminopurine, 1mg/l zeatin, 1% sucrose, 0.6% Phytagar and pH adjusted to 5.8). This medium also contains carbenicillin (500mg/l) and kanamycin sulfate (25mg/l). Hypocotyl explants are subcultured onto fresh shoot induction medium every two weeks.

Shoots regenerate from the hypocotyl calli after one to three months. Green shoots at least lcm tall are excised from the calli and placed on medium containing B5 salts and vitamins, 1% sucrose, carbenicillin (300mg/l), kanamycin sulfate (50mg/l) and 0.6% w/v Phytagar). After 2-4 weeks shoots which remain green are cut at the base and transferred to Magenta boxes containing root induction medium (B5 salts and vitamins, 1% sucrose, 2mg/l indolebutyric acid, 50mg/l kanamycin sulfate and 0.6% Phytagar). Green rooted shoots are tested for thioesterase activity.

### Arabidposis Transformation

Transgenic *Arabidopsis* thaliana plants may be obtained by *Agrobacterium*-mediated transformation as described by Valverkens *et al*., (*Proc*. *Nat*. *Acad*. *Sci.* (1988) *85*:5536-5540). Constructs are transformed *into Agrobacterium* cells, such as of strain EHA101 (Hood *et al*., *J.* Bacteriol (1986) *168*:1291-1301), by the method of Holsters *et al*. (*Mol*. *Gen. Genet.* (1978) *163*:181-187).

### Peanut Transformation

DNA sequences of interest may be introduced as expression cassettes, comprising at least a promoter region, a gene of interest, and a termination region, into a plant genome via particle bombardment as described in European Patent Application 332 855 and in co-pending application USSN 07/225,332, filed July 27, 1988.

Briefly, tungsten or gold particles of a size ranging from 0.5µM-3µM are coated with DNA of an expression cassette. This DNA may be in the form of an aqueous mixture or a dry DNA/particle precipitate.

Tissue used as the target for bombardment may be from cotyledonary explants, shoot meristems, immature leaflets, or anthers.

The bombardment of the tissue with the DNA-coated particles is carried out using a Biolistics™ particle gun (Dupont; Wilmington, DE). The particles are placed in the barrel at variable distances ranging from 1cm-14cm from the barrel mouth. The tissue to be bombarded is placed beneath the stopping plate; testing is performed on the tissue at distances up to 20cm. At the moment of discharge, the tissue is protected by a nylon net or a combination of nylon nets with mesh ranging from 10µM to 300µM.

Following bombardment, plants may be regenerated following the method of Atreya, *et al*., (*Plant Science Letters* (1984) *34*:379-383). Briefly, embryo axis tissue or cotyledon segments are placed on MS medium (Murashige and Skoog, *Physio. Plant.* (1962) *15*:473) (MS plus 2.0 mg/l 6-benzyladenine (BA) for the cotyledon segments) and incubated in the dark for 1 week at 25 ± 2°C and are subsequently transferred to continuous cool white fluorescent light (6.8 W/m²). On the 10th day of culture, the plantlets are transferred to pots containing sterile soil, are kept in the shade for 3-5 days are and finally moved to greenhouse.

The putative transgenic shoots are rooted. Integration of exogenous DNA into the plant genome may be confirmed by various methods know to those skilled in the art.

C. Transgenic plants transformed with thioesterase constructs are analyzed for thioesterase activity and fatty acid and triglyceride compositions.

*Arabidopsis* seeds from selfed transgenic *A. thaliana* plants transformed with pCGN3816 and pCGN3821 are analyzed for 12:0 and 14:0 acyl-ACP thioesterase activities. Developing seeds are extracted with thioesterase assay buffer (Example 1) and the soluble fraction assayed. Transgenic seeds show significant increase of 12:0 thioesterase activity over the controls. Also, the 14:0-ACP hydrolysis increases, but at a smaller scale, in agreement with enzyme specificity data from transformed *E*. *coli*.

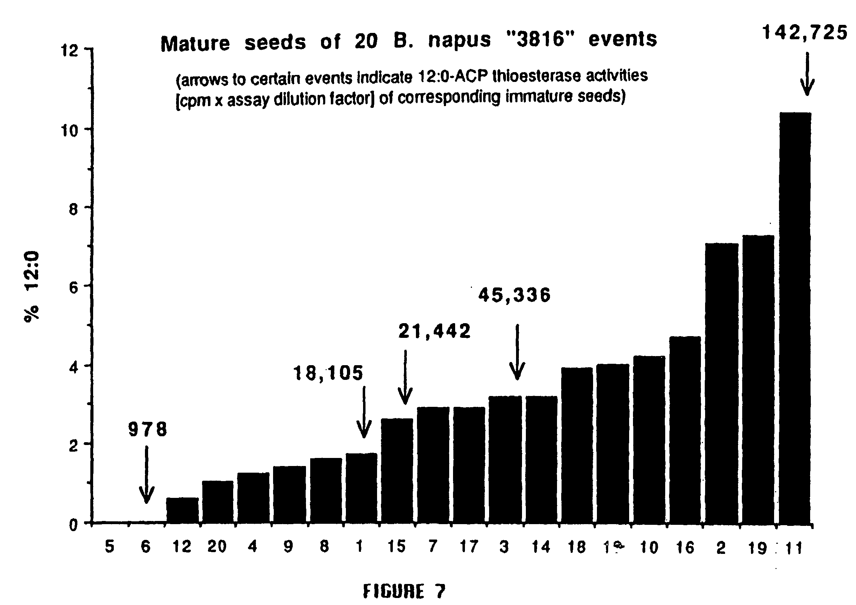

Total fatty acid analysis of mature *A. thaliana* seeds reveals up to 5% laurate in plants transformed with the above described constructs, as compared to 0% laurate as measured in control plant seeds. Figure 7 demonstrates that the percent laurate directly correlates with lauroyl thioesterase activity in transgenic seeds. Also, the myristate content in transgenic seeds increases from 0.1% (control) up to 0.7% in the highest expressers and also correlates with the myristoyl thioesterase activity. Triglyceride analysis by thin-layer chromatography shows that the laurate detected by total fatty acid analysis is present in the neutral lipids fraction, evidence that the laurate is incorporated (esterified) into triglycerides.

Mature seeds from *A. thaliana* plants transformed with pCGN3828 are analyzed for total fatty acids essentially as described by Browse *et al*. (*Anal*. *Biochem*. (1986) *152*:141-145) as described in detail in Example 16. These studies reveal at least one plant, 3828-13, whose seeds contain up to approximately 17% by weight (23.5 mole percent) laurate. Mature seeds from this transformed plant are subjected to a pancreatic lipase digestion protocol (Brockerhoff (1975) *Meth. Enzymol.* 35:315-325) to distinguish acyl compositions of the sn-2 and sn-1+3 (combined) positions. Preliminary results from these analyses are as follows:

| | | |
|---|---|---|
| sn-1+2+3 | (methanolysis) | 17.8% C12 |
| sn-2 | (lipase digestion) | 2.9% C12 |
| sn-1+3 | (calculated from above) | 25.3% C12 |
| sn-1+3 | (lipase digestion) | 21.9% C12. |

These preliminary results suggest that medium-chain fatty acids are efficiently incorporated into the sn-1 and/or sn-3 positions of the triglyceride molecule.

A total of 26 pCGN3828-transformed *Arabidopsis* plants were tested for 12:0-ACP thioesterase activity, with seven testing positive. The presence of "transformants" that are negative for laurate expression is not surprising as the *Arabidopsis* transformation method does not include selection at the rooting stage. Thus, the laurate negative plants would be expected to include non-transformed "escapes," as well as transformed plants which are not expressing the bay thioesterase gene. Analysis of mature seeds (100-seed pools) from these seven positive plants shows that the positive plants contain significant amounts of 12:0, which is absent from controls. The amounts of 12:0 ranged from 2.1 to 23.5 mole percent and approximately correlate with the thioesterase activity. The total fatty acid contents of the seeds are within the range typically seen in *Arabidopsis*, suggesting that the 12:0 deposition does not adversely affect oil yield. No obvious effects on seed development or morphology are observed. Lipid class analysis (TLC) demonstrates that the triglyceride fraction contains the same proportion of laurate as the total extractable fatty acids, i.e. at these levels the 12:0 is readily incorporated into triglyceride.

A small amount of 14:0 also accumulates in transgenic *Arabidopsis* seeds. The ratio of 12:0 to 14:0 fatty acids in the seeds (6-8) is similar to the ratio of in vitro thioesterase activities on 12:0-ACP and 14:0-ACP. The near-constant ratio between the 12:0 and 14:0 products presumably reflects the specificity of the bay thioesterase towards 12:0-ACP and 14:0-ACP, and suggests that the enzyme function in vivo in the transgenic seeds by direct action on similarly sized pools of 12:0-ACP and 14:0-ACP. The bay thioesterase appears to have no significant action on 10:0-ACP *in vitro* and only a minor trace of 10:0 is detected in the transgenic seeds.

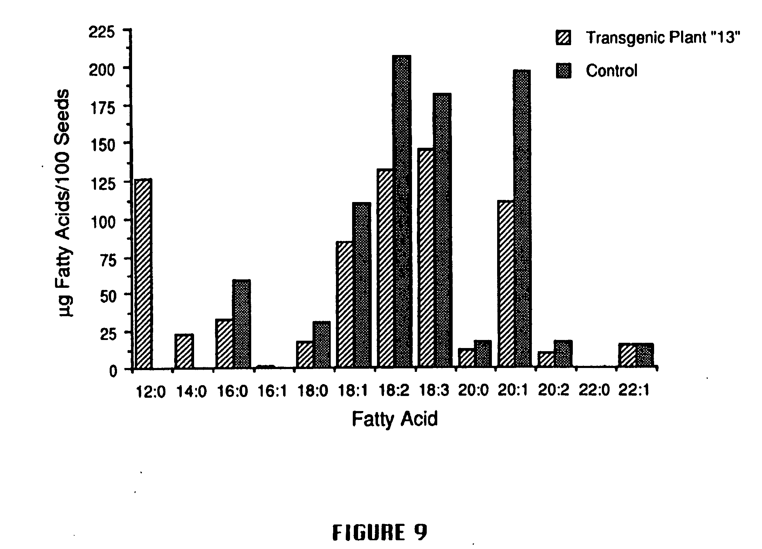

Additional studies were conducted to determine if the medium-chains were synthesized at the expense of all, or only some, of the "native" *Arabidposis* fatty acids. The average fatty acid composition of 100 mature seeds from a control Arabidopsis plant were compared with that from transgenic plant 3828-13. The results of these studies are shown in Figure 9. The differences in 12:0 and 14:0 contents of the two plants are clear, but differences in the contents of other fatty acids as a result of medium-chain production are more difficult to identify. The total fatty acid contents varied considerably between *Arabidopsis* plants, making comparisons of absolute fatty acid levels very difficult. Expression of the data in percentage terms (total fatty acids = 100) to eliminate these differences created further difficulties with interpretation.

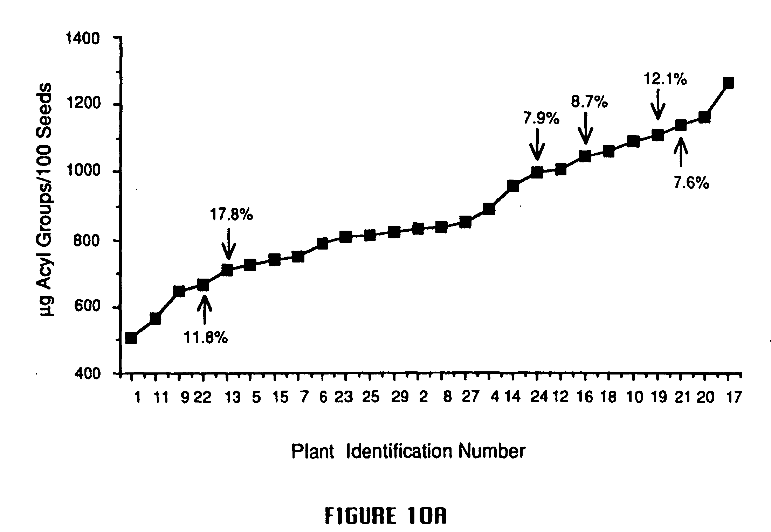

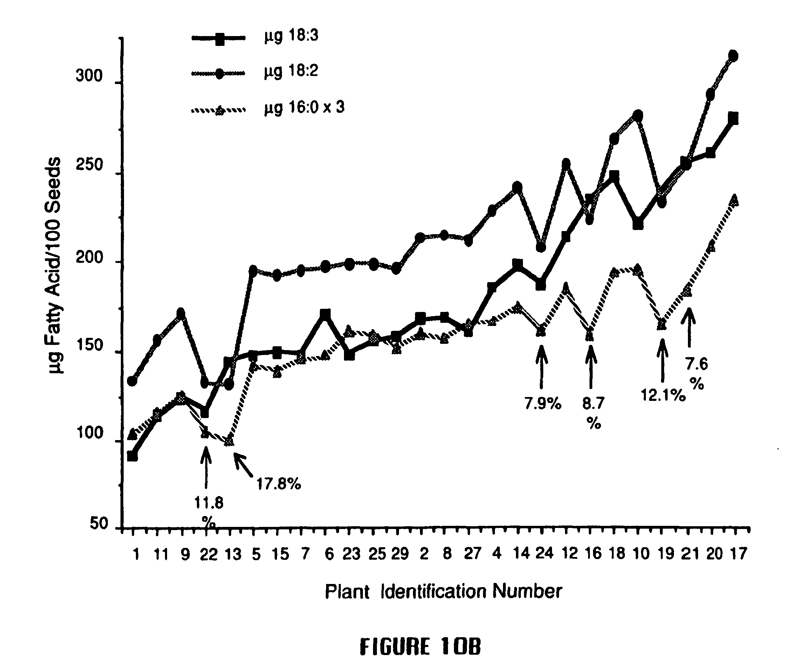

Thus, a way to distinguish unique fatty acid compositions from typical inter-plant variation was devised as follows. The total fatty acid contents of mature (T2) seeds from the 26 T1 *Arabidopsis* plants were arranged in increasing order, and produced a smooth spread of values as shown in Figure 10A. The six highest laurate producers are indicated by arrows, along with the corresponding weight % 12:0 data. There appears to be no relationship between the levels of 12:0 production and total fatty acid content. In Figure 10B the data are shown ordered in the same way, but for three fatty acids individually. The data for 18:2 and 16:0 also formed a smooth line, except for the positive events in which laurate accumulated. In those instances the contents of 18:2 and 16:0 were noticeably below the overall trend, showing that 12:0 was produced in those seeds at the expense of 18:2 and 16:0. This was also true for 18:1, 20:1, and 20:2. The only major fatty acid constituent to be relatively unaffected by 12:0 production was 18:3, as shown in Figure 15B, although low-18:3 controls can be found, for example in plant 10.

Seeds from *Brassica napus* plants transformed with pCGN3816 are also analyzed for total fatty acids as described above. Analysis of single segregating seeds from T2 transformed plants reveals levels of C12:0 ranging from zero to 14.5%, as compared to zero percent in seeds from untransformed control plants. C12:0 levels correlate to C12:0-ACP thioesterase activities in corresponding immature seeds, as demonstrated in Figure 7. In addition, C14:0 is also detected in these seeds at levels correlating with those of the C12:0, although C14:0 levels are lower.

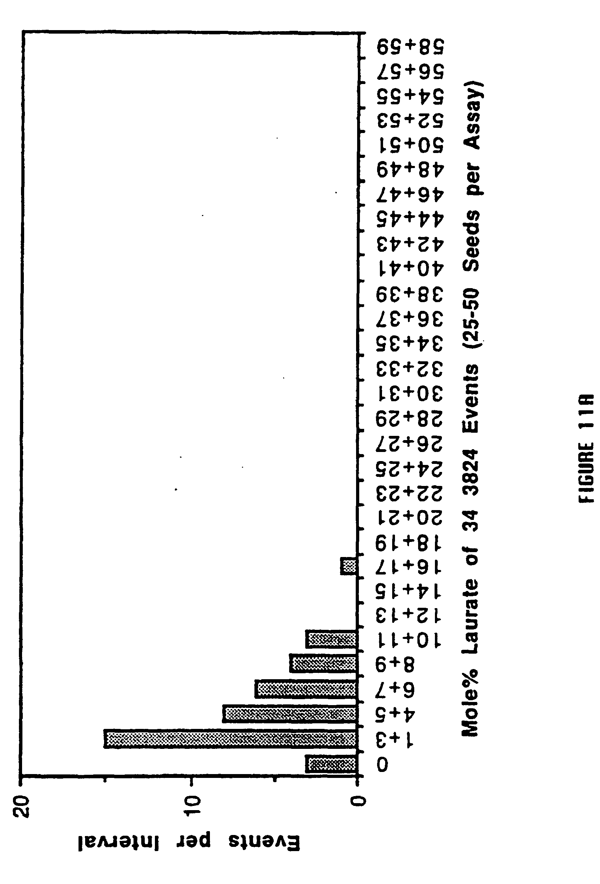

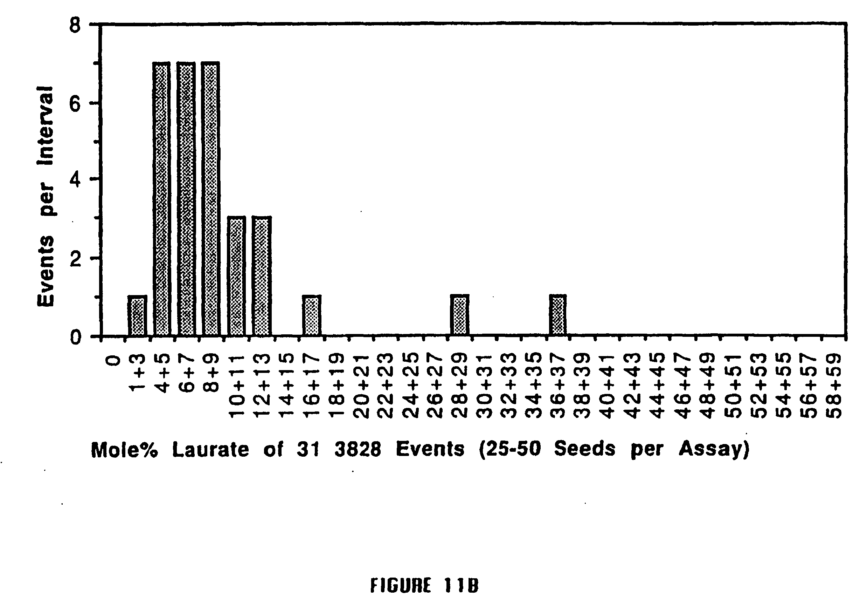

Transformed *Brassica* napus plants containing the pCGN3824 (napin/thioesterase) and pCGN3828 (napin/thioesterase/napin) constructs were analyzed to determine seed fatty acid composition. Pooled seeds from 34 plants transformed with pCGN3824 and 31 plants transformed with pCGN3828 were analyzed (25-50 seeds per assay) to determine the ranges of laurate levels in the seeds. The results of these analyses, presented as the number of transgenic events having a given percentage of laurate, are presented in Figure 11A and 11B. The pCGN3824-transformants had laurate contents ranging from 0-11 mole percent, with the exception of a single plant whose seeds contained 17 mole percent laurate. The pCGN3828 construct plants had laurate contents ranging from 1-17 mole percent, with two representatives outside this range having 37 mole percent laurate (plant 3828-23) and 27 mole percent laurate (plant 3828-35). In addition, the seed oils of these plants also have smaller amounts of C14:0 fatty acids, corresponding to approximately 16% of the laurate levels. Trace levels of C10:0 are also observed, typically at 1% of the laurate level. Additional pCGN3828-transformants are also being analyzed to identify plants having even higher laurate contents.

Half-seed analysis is also used to determine laurate levels in mature seeds from transformed plants. For half-seed analysis, seeds are placed on a moistened (2-3ml water) filter paper disc in a Petri dish which is sealed and left in the dark for 20 to 48 hours at room temperature or 30°C. Germinated seeds have 2-5mm radicles protruding from the seed coats. Fine forceps are used to remove each seedling from its coat and tease away the outer cotyledon. Dissected cotyledons are placed in 4ml vials and dried for 2-12 hours in a 110°C oven prior to fatty acid analysis. The dissected seedlings are planted directly into potting soil in 12-pack containers, misted, covered with transparent plastic lids, placed in a growth chamber at 22°C in 150-200 microEinsteins m⁻²s⁻¹ light intensity with a 16h/8h photoperiod, and allowed to grow to produce T2 (second generation transformants) plants. Alternatively, half-seed analysis may be conducted using a chipped portion of a mature seed. Seeds are held under a dissecting scope and a chip of approximately 30% of the seed is removed, avoiding the embryonic axis. The seed chip is used for fatty acid analysis by GC, and the remaining seed portion is germinated in water for 5-7 days in a microtiter dish, transferred to soil, and grown to produce T2 seed.

The laurate content of 144 assayed pCGN3828-35 half seeds ranged from 4 to 42 mole percent. The laurate content of 214 assayed pCGN3828-23 half seeds ranged from 12 to 50 mole percent. No seeds that were analyzed from either the pCGN3828-23 or pCGN3828-35 plants had zero laurate, indicating that these transformants have three or more thioesterase inserts in their genome. In addition, analyses using approximately 60 half-seeds of the pCGN3828-transformants having 10-20 mole % laurate in their seeds indicates that these plants have 1-2 insertions of the bay thioesterase gene.

To examine the fate of the laurate in transgenic *Brassica napus* seeds, the fatty acid compositions of different lipid classes extracted from mature transgenic seeds of two transgenic plants, pCGN3828-23 and pCGN3828-7, were examined. TLC analysis of the phospholipids indicates that nearly 100% of the laurate is in the TAG fraction. Analyses of the acyl compositions of the sn-2 and sn-1+3 positions of the TAG are conducted using the pancreatic lipase protocol (Brockerhoff (1975), *supra*). Ideally with this protocol, the lipase cleaves fatty acids from the sn-1 and sn-3 positions, and not from the sn-2 position. Thus, the fatty acids in the resulting mono-glyceride are presumed to be those in the sn-2 position. Initial studies of TAG in the laurate transformants with this method indicate that C12:0 fatty acids are not incorporated into the sn-2 position. However, it is noted that those previously attempting to study TAG having shorter-chain fatty acids by this method (Entressangles *et al*. (1964) *Biochem. Biophys. Acta 84*:140-148), reported that shorter-chain fatty acids located at the sn-2 position were quickly hydrolyzed during such a digestion, which the authors reported to be the result of a spontaneous migration of internal shorter-chain fatty acids towards outer positions in diglycerides and monoglycerides.

Additional analyses of transformed plants containing the pCGN3828 construct are conducted to further characterize the expression of bay thioesterase in these plants. The extractable C12:0 thioesterase activity in developing seeds of pCGN3828-23 transformants is measured and is determined to be considerably higher than the endogenous 18:1 thioesterase activity. In view of the high bay thioesterase activity in transgenic plants, additional factors are being investigated for optimization of laurate production.

The presence of the processed (34kD) bay thioesterase in transformed 3828-23 plants is investigated by Western analysis of a developmental time course of seeds from this plant. Experiments are conducted using polyclonal antibody to bay thioesterase and a biotin labeled second antibody. These studies indicate that a major seed storage protein in *Brassica* migrates with the same mobility as the bay thioesterase, causing non-specific background staining. However, a band of approximately 42kD apparent molecular weight which reacts with the bay Ab is detected in transformed laurate producing plants. This apparent molecular weight is consistent with that of the unprocessed bay thioesterase.

Alternate Western detection methods are under study to reduce the non-specific background staining. For example, a second antibody method where the second antibody is coupled to alkaline phosphatase, results in reduced background staining. Accumulation of bay thioesterase is detectable at low levels at day 24 after pollination, with strong signals observed in seeds from days 30-40 after pollination. Initial results suggest that most of the signal is the 42kD unprocessed preprotein, with only 10-20% of the thioesterase antigen migrating at 34kD. These studies suggest that the unusual transit peptide of the bay thioesterase may result in non-optimal plastid targeting in *Brassica*.

RNA analysis of the above developmental time course seed samples shows that the napin-driven bay thioesterase mRNA accumulates with the same kinetics as the total endogenous napin message, with peak transcription in the 27-50 day range. Thus, the bay thioesterase activity lags behind the onset of storage oil synthesis by about 5-7 days, and earlier expression of the bay thioesterase may make a significant impact on total laurate levels in mature seeds. Northern analysis of ACP and stearoyl-ACP desaturase transcripts in the above seed samples indicates that the native transcripts of these genes accumulate 3-5 days earlier than the bay thioesterase transcript produced by the napin promoter. These data suggest that the ACP and stearoyl-ACP desaturase gene promoters may be useful for earlier expression of the bay thioesterase gene. Cloning of a cDNA for a *Brassica rapa* stearoyl-ACP desaturase and a promoter region for *B. rapa* ACP have been described (Knutzon *et al*. (1992) *Proc. Nat. Acad. Sci. 89*:2624-2628; Scherer *et al*. (1992) *Plant Mol*. *Biol. 18*:591-594).

Transformed *Arabidopsis* plants which contain a construct (pCGN3836) having the 1.2kb bay thioesterase gene fragment positioned for expression from an approximately 1.5 kb region of the *B. rapa* ACP promoter, and approximately 0.3kb of a napin 3' regulatory region, have been obtained. Initial analysis of the seeds from the pCGN3836-transformed plants for laurate content, indicates that laurate does not accumulate to detectable levels in these seeds. However, it is possible that when expression timing and targeting of bay thioesterase are optimized in transgenic *Brassica* seeds a small amount of thioesterase will make a great deal of laurate, as appears to occur in bay, and a lower level of expression of bay thioesterase may be sufficient.

### Example 4 - Transgenic Plants

Plants transformed with thioesterase constructs are analyzed for thioesterase activity and fatty acid and triglyceride compositions.

### A. Arabidopsis

*Arabidopsis* seeds from selfed transgenic *A*. *thaliana* plants transformed with pCGN3816 and pCGN3821 are analyzed for 12:0 and 14:0 acyl-ACP thioesterase activities. Developing seeds are extracted with thioesterase assay buffer (Pollard, *et al*, *supra*) and the soluble fraction assayed. Transgenic seeds show significant increase of 12:0 thioesterase activity over the controls. Also, the 14:0-ACP hydrolysis increases, but at a smaller scale, in agreement with enzyme specificity data from transformed E. coli.

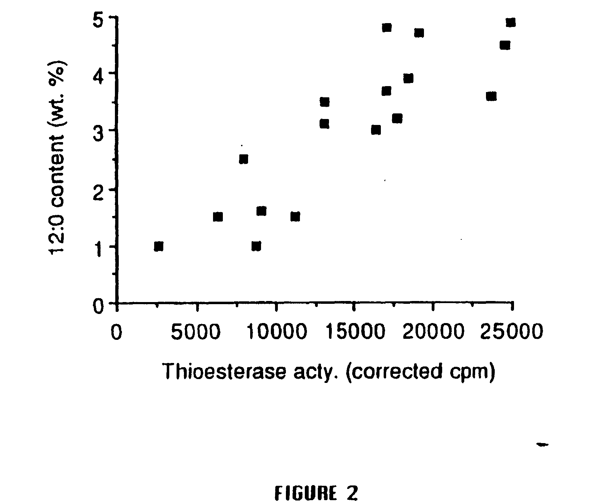

Total fatty acid analysis of mature A. thaliana seeds reveals up to 5% laurate in plants transformed with the above described constructs, as compared to 0% laurate as measured in control plant seeds. Figure 2 demonstrates that the percent laurate directly correlates with lauroyl thioesterase activity in transgenic seeds. Also, the myristate content in transgenic seeds increases from 0.1% (control) up to 0.7% in the highest expressers and also correlates with the myristoyl thioesterase activity. Triglyceride analysis by thin-layer chromatography (TLC) shows that the laurate detected by total fatty acid analysis is present in the neutral lipids fraction, evidence that the laurate is incorporated (esterified) into triglycerides.

Mature seeds from *A*. *thaliana* plants transformed with pCGN3828 are analyzed for total fatty acids by GC essentially as described by Browse *et al*. *(Anal. Biochem.* (1986) *152*:141-145) as described in detail in Example 2. These studies reveal at least one plant, 3828-13, whose seeds contain up to approximately 17% by weight (23.5 mole percent) laurate. Mature seeds from this transformed plant are subjected to a pancreatic lipase digestion protocol (Brockerhoff (1975) *Meth. Enzymol. 35*:315-325) to distinguish acyl compositions of the sn-2 and sn-1+3 (combined) positions. Preliminary results from these analyses are as follows:

| | | |
|---|---|---|
| sn-1+2+3 | (methanolysis) | 17.8% C12 |
| sn-2 | (lipase digestion) | 2.9% C12 |
| sn-1+3 | (calculated from above) | 25.3% C12 |
| sn-1+3 | (lipase digestion) | 21.9% C12. |

These preliminary results suggest that medium-chain fatty acids are efficiently incorporated into the sn-1 and/or sn-3 positions of the triglyceride molecule. (Further discussion of this technique is provided below.)

In a different experiment, out of 26 pCGN3828-transformed *Arabidopsis* plants tested for 12:0-ACP thioesterase activity, seven tested positive. The presence of "transformants" that are negative for laurate expression is not surprising as the *Arabidopsis* transformation method does not include selection at the rooting stage. Thus, the laurate negative plants would be expected to include non-transformed "escapes," as well as transformed plants which are not expressing the bay thioesterase gene. Analysis of mature seeds (100-seed pools) from these seven positive plants shows that the positive plants contain significant amounts of 12:0, which is absent from controls. The amounts of 12:0 ranged from 2.1 to 23.5 mole percent and approximately correlate with the thioesterase activity. The total fatty acid contents of the seeds are within the range typically seen in *Arabidopsis,* suggesting that the 12:0 deposition does not adversely affect oil yield. No obvious effects on seed development or morphology are observed. Lipid class analysis (TLC) demonstrates that the triglyceride fraction contains the same proportion of laurate as the total extractable fatty acids, i.e. at these levels the 12:0 is readily incorporated into triglyceride.

A small amount of 14:0 also accumulates in transgenic *Arabidopsis* seeds. The ratio of 12:0 to 14:0 fatty acids in the seeds is similar to the ratio of in vitro thioesterase activities on 12:0-ACP and 14:0-ACP. The near-constant ratio between the 12:0 and 14:0 products presumably reflects the specificity of the bay thioesterase towards 12:0-ACP and 14:0-ACP, and suggests that the enzyme function in vivo in the transgenic seeds by direct action on similarly sized pools of 12:0-ACP and 14:0-ACP. The bay thioesterase appears to have no significant action on 10:0-ACP *in vitro* and only a minor trace of 10:0 is detected in the transgenic seeds.

Additional studies were conducted to determine if the medium-chains were synthesized at the expense of all, or only some, of the "native" *Arabidopsis* fatty acids. The average fatty acid composition of 100 mature seeds from a control *Arabidopsis* plant were compared with that from transgenic plant 3828-13. The results of these studies are shown in Figure 9. The differences in 12:0 and 14:0 contents of the two plants are clear, but differences in the contents of other fatty acids as a result of medium-chain production are more difficult to identify. The total fatty acid contents varied considerably between *Arabidopsis* plants, making comparisons of absolute fatty acid levels very difficult. Expression of the data in percentage terms (total fatty acids = 100) to eliminate these differences created further difficulties with interpretation.

Thus, a way to distinguish unique fatty acid compositions from typical inter-plant variation was devised as follows. The total fatty acid contents of mature (T2) seeds from the 26 T1 *Arabidopsis* plants were arranged in increasing order, and produced a smooth spread of values as shown in Figure 10A. The six highest laurate producers are indicated by arrows, along with the corresponding weight percent 12:0 data. There appears to be no relationship between the levels of 12:0 production and total fatty acid content. In Figure 10B the data are shown ordered in the same way, but for three fatty acids individually. The data for 18:2 and 16:0 also formed a smooth line, except for the positive events in which laurate accumulated. In those instances the contents of 18:2 and 16:0 were noticeably below the overall trend, showing that 12:0 was produced in those seeds at the expense of 18:2 and 16:0. This was also true for 18:1, 20:1, and 20:2. The only major fatty acid constituent to be relatively unaffected by 12:0 production was 18:3, as shown in Figure 10B, although low-18:3 controls can be found, for example in plant 10.

### B. Brassica

Seeds from *Brassica napus* plants transformed with pCGN3816 are also analyzed for total fatty acids by GC as described above. Analysis of single segregating seeds (T2 seeds) from transformed plants (T1 plants) reveals levels of C12:0 ranging from zero to 14.5%, as compared to zero percent in seeds from untransformed control plants. C12:0 levels correlate to C12:0-ACP thioesterase activities in corresponding immature seeds, as demonstrated in Figure 7. In addition, C14:0 is also detected in these seeds at levels correlating with those of the C12:0, although C14:0 levels are lower.

Minor modifications may be made to the GC temperature program used for analysis of laurate-containing TAG. An additional useful temperature cycle is as follows: 160°C for 3 minutes, followed by a 5 degrees per minute temperature ramp to final temperature of 240°C, which is held for 6 minutes; this results in a total run time of 26 minutes.

Transformed *Brassica napus* plants containing the pCGN3824 (napin/thioesterase) and pCGN3828 (napin/thioesterase/napin) constructs were analyzed to determine seed fatty acid composition. Pooled seeds from 34 plants transformed with pCGN3824 and 31 plants transformed with pCGN3828 were analyzed (25-50 seeds per assay) to determine the ranges of laurate levels in the seeds. The results of these analyses, presented as the number of transgenic events having a given percentage of laurate, are presented in Figure 11. The pCGN3824-transformants had laurate contents ranging from 0-11 mole percent, with the exception of a single plant whose seeds contained 17 mole percent laurate. The pCGN3828 construct plants had laurate contents ranging from 1-17 mole percent, with two representatives outside this range having 37 mole percent laurate (plant 3828-23) and 27 mole percent laurate (plant 3828-35). It is noted that in addition to containing laurate, the seed oils of these plants also have smaller amounts of C14:0 fatty acids, corresponding to approximately 16% of the laurate levels.

Half-seed analysis is also used to determine laurate levels in mature seeds from transformed plants. For half-seed analysis, seeds are placed on a moistened (2-3ml water) filter paper disc in a Petri dish which is sealed and left in the dark for 20 to 48 hours at room temperature or 30°C. Germinated seeds have 2-5mm radicles protruding from the seed coats. Fine forceps are used to remove each seedling from its coat and tease away the outer cotyledon. Dissected cotyledons are placed in 4ml vials and dried for 2-12 hours in a 110°C oven prior to fatty acid analysis. The dissected seedlings are planted directly into potting soil in 12-pack containers, misted, covered with transparent plastic lids, placed in a growth chamber at 22°C in 150-200 microEinsteins m⁻²s⁻¹ light intensity with a 16h/8h photoperiod, and allowed to grow to produce T2 (second generation transformants) plants. Alternatively, half-seed analysis may be conducted using a chipped portion of a mature seed. Seeds are held under a dissecting scope and a chip of approximately 30% of the seed is removed, avoiding the embryonic axis. The seed chip is used for fatty acid analysis by gas chromatography, and the remaining seed portion is germinated in water for 5-7 days in a microtiter dish, transferred to soil, and grown to produce T2 plants. A chart providing fatty acid composition as mole percent of total fatty acids of 15 representative pCGN3828-23 half-seeds is shown in Table 4A. Similar data from single seeds collected from non-transformed regenerated control plants are shown in Table 4B. Data are from GC half-seed analysis as described above.

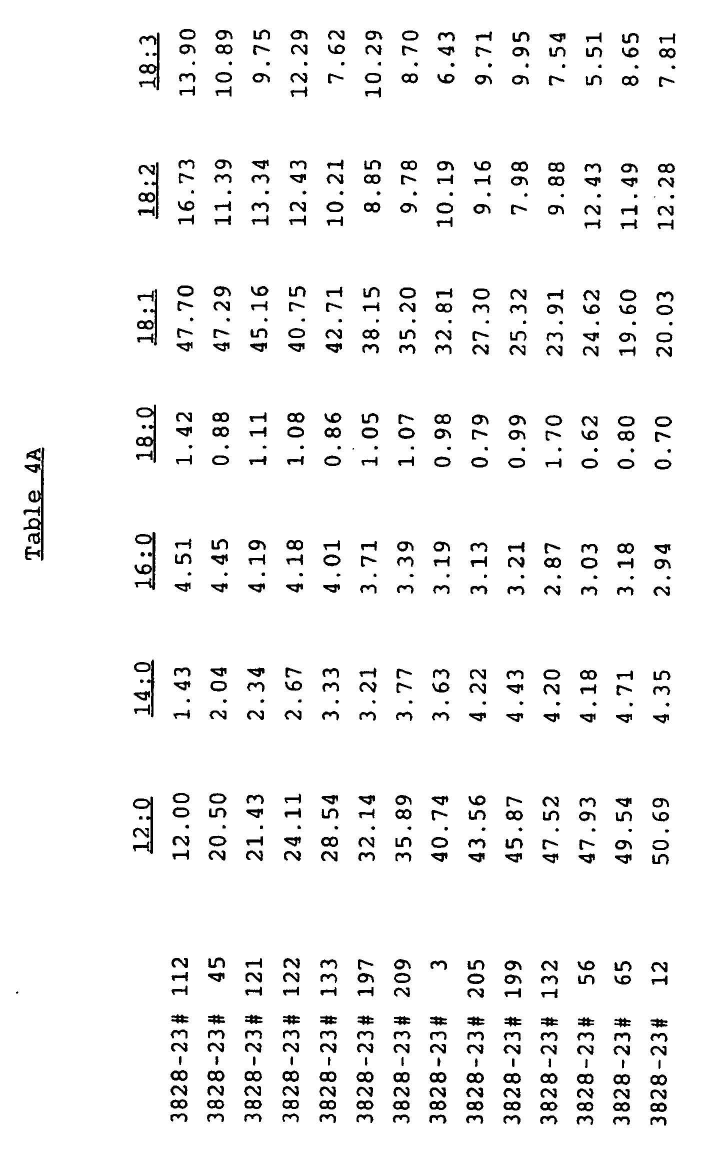

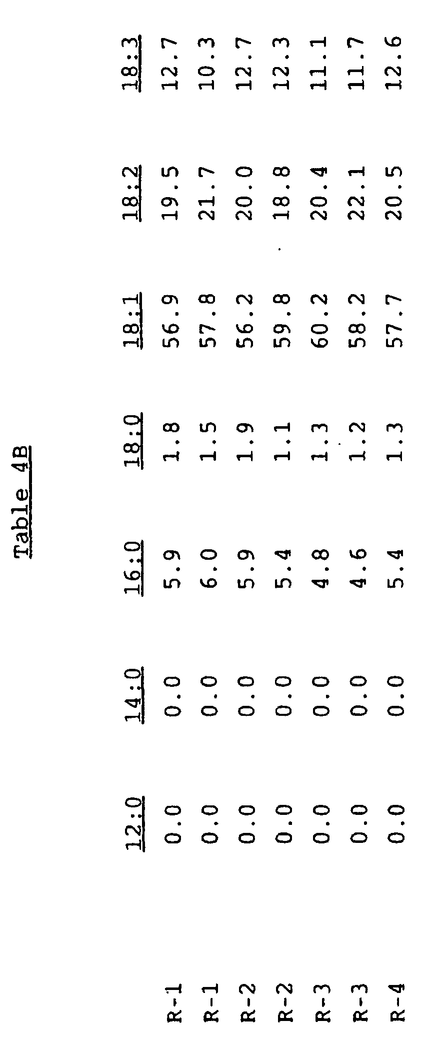

The laurate content of 144 assayed pCGN3828-35 half seeds (T2 seed obtained from a T1 plant) ranged from 4 to 42 mole percent. The laurate content of 214 assayed pCGN3828-23 half seeds ranged from 12 to 50 mole percent. No seeds that were analyzed from either the pCGN3828-23 or pCGN3828-35 plants had zero laurate which statistically indicates that these transformants have three or more thioesterase inserts in their genome. Analysis of seed produced from the T2 generation will further confirm this result. In addition, analyses using approximately 60 half-seeds of the pCGN3828-transformants having 10-20 mole percent laurate in their seeds indicates that these plants have 1-2 insertions of the bay thioesterase gene.

To examine the fate of the laurate in transgenic Brassica napus seeds, the fatty acid compositions of different lipid classes extracted from mature transgenic seeds of two transgenic plants, pCGN3828-23 and pCGN3828-7, were examined. TLC analysis of the phospholipids indicates that nearly 100% of the laurate is in the triacylglyceride (TAG) fraction. Analyses of the acyl compositions of the sn-2 and sn-1+3 positions of the TAG are conducted using the pancreatic lipase protocol (Brockerhoff (1975), supra). Ideally with this protocol, the lipase cleaves fatty acids from the sn-1 and sn-3 positions, and not from the sn-2 position. Thus, the fatty acids in the resulting mono-glyceride are presumed to be those in the sn-2 position. Initial studies of TAG in the laurate transformants with this method indicate that C12:0 fatty acids are not incorporated into the sn-2 position. However, it is noted that those previously attempting to study TAG having shorter-chain fatty acids by this method (Entressangles *et al*. (1964) *Biochim. Biophys. Acta 84*:140-148), reported that shorter-chain fatty acids located at the sn-2 position were quickly hydrolyzed during such a digestion, which the authors reported to be the result of a spontaneous migration of internal shorter-chain fatty acids towards outer positions in diglycerides and monoglycerides.

Additional analyses of transformed plants containing the pCGN3828 construct are conducted to further characterize the expression of bay thioesterase in these plants. The extractable C12:0 thioesterase activity in developing seeds of pCGN3828-23 transformants is measured and is determined to be considerably higher than the endogenous 18:1 thioesterase activity. In view of the high bay thioesterase activity in transgenic plants, additional factors are being investigated for optimization of laurate production.

The presence of the processed (34kD) bay thioesterase in transformed 3828-23 plants is investigated by Western analysis of a developmental time course of seeds from this plant. Experiments are conducted using polyclonal antibody to bay thioesterase and a biotin labeled second antibody. These studies indicate that a major seed storage protein in Brassica migrates with the same mobility as the bay thioesterase, causing non-specific background staining. However, a band of approximately 42kD apparent molecular weight which reacts with the bay antibody is detected in transformed laurate producing plants. This apparent molecular weight is consistent with that of the unprocessed bay thioesterase.

Alternate Western detection methods are under study to reduce the non-specific background staining. For example, a second antibody method where the second antibody is coupled to alkaline phosphatase, results in reduced background staining. Accumulation of bay thioesterase is detectable at low levels at day 24 after pollination, with strong signals observed in seeds from days 30-40 after pollination. Initial results suggest that most of the signal is the 42kD unprocessed preprotein, with only 10-20% of the thioesterase antigen migrating at 34kD. These studies suggest that the unusual transit peptide of the bay thioesterase may result in non-optimal plastid targeting in *Brassica*.

RNA analysis of the above developmental time course seed samples shows that the napin-driven bay thioesterase mRNA accumulates with the same kinetics as the total endogenous napin message, with peak transcription in the 27-50 day range. Thus, the bay thioesterase activity lags behind the onset of storage oil synthesis by about 5 - 7 days, and earlier expression of the bay thioesterase may make a significant impact on total laurate levels in mature seeds. Northern analysis of ACP and stearoyl-ACP desaturase transcripts in the above seed samples indicates that the native transcripts of these genes accumulate 3-5 days earlier than the bay thioesterase transcript produced by the napin promoter. These data suggest that the ACP and stearoyl-ACP desaturase gene promoters may be useful for earlier expression of the bay thioesterase gene. Cloning of a cDNA for a Brassica rapa stearoyl-ACP desaturase and a promoter region for *B*. *rapa* ACP have been described (Knutzon *et al*. (1992) *Proc. Nat. Acad. Sci*. *89*:2624-2628; Scherer *et al*. (1992) *Plant Mol*. *Biol*. *18*:591-594).

Transformed *Arabidopsis* plants which contain a construct (pCGN3836) having the 1.2kb bay thioesterase gene fragment positioned for expression from an approximately 1.5 kb region of the *B. rapa* ACP promoter, and approximately 0.3kb of a napin 3' regulatory region, have been obtained. Initial analysis of the seeds from the pCGN3836-transformed plants for laurate content, indicates that laurate does not accumulate to detectable levels in these seeds. However, it is possible that when expression timing and targeting of bay thioesterase are optimized in transgenic *Brassica* seeds a small amount of thioesterase will make a great deal of laurate, as appears to occur in bay, and a lower level of expression of bay thioesterase may be sufficient.

### Example 5 - Obtaining Other Plant Thioesterases

### A. Additional Sources of Plant Thioesterases

In addition to the Bay and safflower thioesterases identified in previous Examples, other plants are sources of desirable thioesterases which have varying specificities with respect to fatty acyl chain length and/or degree of saturation. Such additional plant thioesterases may be identified by analyzing the triacylglyceride composition of various plant oils and the presence of a specific thioesterase confirmed by assays using the appropriate acyl-ACP substrate.

Other plants which may have desirable thioesterase enzymes include elm *(Ulmaceae)* and camphor *(Cinnamomum camphora*). A significant percentage of 10:0 fatty acids are detected in elm seeds, and both 10:0 and 12:0 fatty acids are prominent in seeds from camphor. Results of biochemical assays to test for thioesterase activity in developing embryos from camphor and elm are presented below in Table 5.

**Table 5**

| Substrate | Activity | |
|---|---|---|
| | (mean cpm in ether extract) | |
| | elm | camphor |
| 8:0-ACP | 84 | 0 |
| 10:0-ACP | 2199 | 465 |
| 12:0-ACP | 383 | 1529 |
| 14:0-ACP | 1774 | 645 |
| 16:0-ACP | 3460 | 940 |
| 18:1-ACP | 3931 | 3649 |

With elm, a peak of thioesterase activity is seen with the C10:0-ACP substrate, in addition to significant activity with longer-chain substrates. This evidence suggests that a thioesterase with specific activity towards C10:0-ACP substrate is present in elm embryos. Significant activity towards C12:0-ACP substrate is detected in camphor extracts. In addition, camphor extracts demonstrate greater activity towards C10:0-ACP substrates than do similar extracts from bay embryos. This evidence suggests that a medium-chain acyl-ACP thioesterase having specificity towards C10:0-ACP and C12:0-ACP substrates is present in camphor embryos.

In a like fashion, longer chain fatty acyl thioesterase (C16 or C18) can also be obtained. For example, a significant percentage (45%) of 16:0 fatty acids is found in the tallow layer of the seeds of the Chinese tallow tree (*Sapium sebiferum*) and in the seed oil of cotton (*Gossypium hirsutum*) (Gunstone, Harwood and Padley eds. *The Lipid Handbook*, (1986) Chapman and Hall, Ltd., The University Press, Cambridge).

Approximately 250mg each of developing Chinese tallow tissue, cotton embryos (var. Stoneville 506, day 21 post-anthesis) or *Brassica napus* embryos (cv. Delta, day 28 post-anthesis) are ground to a fine powder in a mortar and pestle under liquid nitrogen and extracted by homogenization in 1 ml 50mM sodium phosphate pH 7.5, 2 mM dithiothreitol, 2 mM sodium ascorbate, 20% v/v glycerol, 1% w/v PVP-10 and 5 mM diethyldithiocarbamate in a glass homogenizer with a motor driven pestle. The homogenate is centrifuged in a microcentrifuge tube for 15 min and aliquots of the supernatant fraction are assayed for thioesterase activity as follows.

Twenty-five µl of a 1/20 dilution of the supernatant in assay buffer (7 mM potassium phosphate, pH 8.0, 20% v/v glycerol, 0.02% w/v Triton X-100, 1 mM dithiothreitol) is added to 70 µl of assay buffer in a glass screw top vial. Fifty pmoles of [¹⁴C]-radiolabeled acyl-substrate are added to start the reaction. The substrates are myristoyl-ACP (14:0-ACP), palmitoyl-ACP (16:0-ACP), stearoyl-ACP (18:0-ACP) or oleoyl-ACP (18:1-ACP) synthesized as described for lauroyl-ACP in Pollard, *et al*., *supra.* Vials are incubated 30 min, 30 C. The reactions are stopped with acetic acid and free fatty acids are extracted with ether by adding 0.5ml 10% (v/v) cold (4°) acetic acid and placing the reaction mixture on ice for a few minutes. The fatty acid product of the hydrolytic enzyme action is extracted away from the unhydrolyzed substrate by adding 2ml diethyl ether and mixing vigorously. The ether is transferred to 5ml scintillation fluid for scintillation counting. Additional ether extracts may be performed to recover remaining traces of product for more accurate quantitation of the activity if desired.

Substrate specificity analysis results for cotton, Chinese tallow and *Brassica* are shown in Table 6.

**Table 6**

| Substrate | Activity | | |
|---|---|---|---|
| | (mean cpm in ether extract) | | |
| | tallow | cotton | *Brassica* |
| 14:0-ACP | 254 | 944 | 180 |
| 16:0-ACP | 1038 | 1542 | 506 |
| 18:0-ACP | 733 | 860 | 500 |
| 18:1-ACP | 2586 | 3667 | 4389 |

A peak of activity is seen with the 16:0-ACP substrate as well as the 18:1-ACP substrate in both cotton and Chinese tallow whereas the *Brassica* seed profile only shows significant activity with the 18:1-ACP. It appears that an acyl-ACP thioesterase with specificity for 16:0 fatty-acyl ACP accounts for the triacylglyceride composition of Chinese tallow and cotton.

Two peaks of thioesterase activity are observed in extracts of cotton embryos chromatographed on heparin-agarose. This chromatography has been shown to separate two different thioesterases, a 12:0-ACP thioesterase and an 18:1 thioesterase from Bay extracts (Pollard, *et al*., *Arch. Biochem*. *Biophys.* (1991) *284*:306-312). Of the two peaks of activity observed from the chromatography of cotton extracts the first has higher 18:1 activity than 16:0 activity and the second peak has higher 16:0 activity than 18:1 activity. The data suggests the presence of two enzymes with distinct specificities in cotton.

In addition, kernel oil of mango (*Mangifera indica*) contains 24-49% stearic acid and 6-18% palmitic acid in triacylglycerols and the oil has been suggested for use as a cocoa butter substitute (Osman, S.M., "Mango Fat", in *New Sources of Fats and Oils,* (1981) eds. Pryde, E.H., Princen, L.H., and Mukherjee, K.D., American Oil Chemists Society). Similarly to the examples described above, a thioesterase with 18:0-ACP specificity can be demonstrated by biochemical assay of embryo extracts.

### B. Isolating Thioesterase Genes

Having obtained sequence (amino acid and DNA) for Bay and safflower thioesterase, similar genes from other plant sources such as those identified above can be readily isolated. In this example, two methods are described to isolate other thioesterase genes: (1) by DNA hybridization techniques using sequences or peptide sequence information from the Bay and safflower thioesterase gene and (2) by immunological cross-reactivity using antibodies to the Bay protein as a probe.

In either of these techniques, cDNA or genomic libraries from the desired plants are required. Many methods of constructing cDNA or genomic libraries are provided for example in Chapter 8 and 9 of Maniatis, *et al*. *(Molecular Cloning: A Laboratory Manual,* Second Edition (1989) Cold Spring Harbor Laboratory, Cold Spring Harbor, New York).

Probes for use in DNA hybridizations to isolate other plant thioesterase genes can be obtained from the Bay and safflower thioesterase gene sequences provided or alternatively by PCR using oligonucleotides from thioesterase peptide sequences.

In this example, a PCR-generated DNA fragment is used as a probe. Northern analysis of embryo RNA from the desired plant species is conducted to determine appropriate hybridization conditions. RNA is electrophoresed in a formaldehyde/agarose gel and transferred to a nylon membrane filter as described by Fourney, *et al*. (*Focus* (1988) Bethesda Research Laboratories/Life Technologies, Inc., *10*:5-7. A ³²P-labeled probe (Random Primed DNA labeling kit, Boehringer Mannheim, Indianapolis, IN) is added to a hybridization solution containing 50% formamide, 6 x SSC (or 6 x SSPE), 5 x Denhardt's reagent, 0.5% SDS, and 100µg/ml denatured salmon sperm DNA fragments.

The hybridization solution containing the labeled probe is incubated with the Northern filter at approximately 40°C for 18 hours or longer to allow hybridization of the probe to homologous (50-80%) sequences. The filter is then washed at low stringency (room temperature to 42°C in about 1X SSC). Hybridization and washing temperatures may be adjusted based on the estimated melting temperature of the probe as discussed in Beltz, *et al*. *(Methods in Enzymology* (1983) *100*:266-285). In further testing the temperature is raised either in the hybridization or washing steps, and/or salt content is lowered to improve detection of the specific hybridizing sequence.

A useful probe and appropriate hybridization and washing conditions having been identified as described above, cDNA libraries are screened using the ³²P-labeled fragment and optimized conditions.

For example, an ∼600bp *Bam*HI/*Xho*I fragment of thioesterase clone pCGN3263 is radio-labeled and used as a heterologous probe to isolate a thioesterase clone from a *B*. *campestris* embryo cDNA library. DNA sequence of a *Brassica* thioesterase cDNA clone is presented in Figure 6. Along with the translated amino acid sequence from the proposed ATG start codon. Additional *Brassica* clones which show some variations in DNA sequence are also being analyzed.

In addition to direct hybridization techniques using heterologous thioesterase genes as probes, PCR techniques may also be used to create probes for hybridization or to generate thioesterase encoding sequences from mRNA or DNA from the desired plant source. For example, a camphor (*Cinnamomum camphora*) thioesterase clone may be isolated using nucleic acid and amino acid sequence information from the bay and safflower thioesterase clones. Homology of the bay thioesterase cDNA clone to RNA isolated from developing camphor embryos is observed by Northern analysis as follows. Total RNA is isolated from 1g of developing camphor embryos by adaptation of the SDS/phenol extraction method described in *Current Protocols in Molecular Biology,* pages 4.3.1-4.3.4 (Ausubel *et al*., eds. (1987); John Wiley & Sons). The grinding buffer for this extraction contains 100mM LiCl, 100mM Tris pH9, 10mM EDTA, 1%SDS and 0.5% β-mercaptoethanol. For extraction from 1g of embryos, 10ml of grinding buffer plus 3ml of phenol equilibrated to pH8 are added to powdered embryos. The homogenization step may be conducted in a mortar instead of with a polytron, as described in the published method, and the heating step which follows homogenization in that method is omitted. Centrifugation, phenol/chloroform extractions of the sample and LiCl precipitation of RNA are as described.

Total RNA (10-20µg) is electrophoresed in a formaldehyde/agarose gel and transferred to a nylon membrane filter as described by Fourney *et al*. (*supra*). A probe for hybridization of the Northern filter is prepared from a *Sal*I digest of pCGN3822, the full length bay thioesterase cDNA by PCR using oligonucleotides to the safflower thioesterase cDNA sequence to generate an approximately 1300bp fragment. The forward primer contains nucleotides 212 to 228 of the safflower thioesterase cDNA sequence (SEQ ID NO:38) and the reverse primer is the complement to nucleotides 1510-1526 of the cDNA sequence. The fragment is gel purified using a Prep-A-Gene DNA purification kit (BioRad; Richmond, CA) and radiolabeled using a Boehringer Mannheim (Indianapolis, IN) random priming labeling kit. The Northern filter is hybridized overnight in 50% formamide, 5X SSC, 50mM sodium phosphate (pH7), 5X Denhardt's solution, 0.1% SDS, 5mM EDTA and 0.1mg/ml denatured DNA at 30°C. The filter is washed twice (15 minutes each wash) in 0.1X SSC, 0.1% SDS. Autoradiography of the hybridized filter reveals a strong hybridization signal to an approximately 1300bp RNA band in the camphor embryo sample. This band is approximately the same size as the bay thioesterase mRNA.

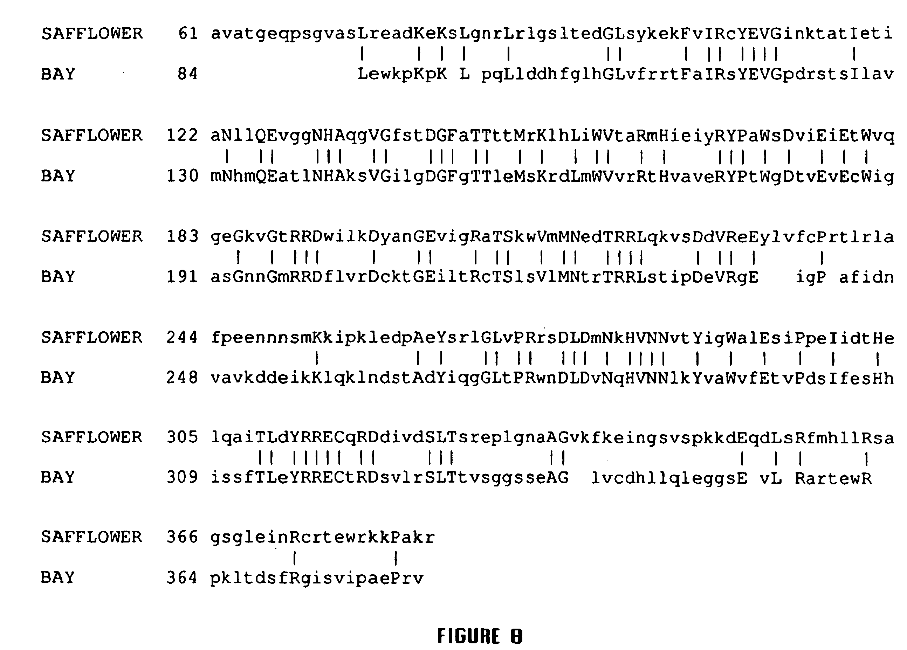

To obtain a fragment of the camphor thioesterase gene, PCR is conducted using oligonucleotides to peptides conserved between the bay and safflower thioesterases. A comparison of the safflower and bay thioesterase translated amino acid sequence is presented in Figure 8.

Polymerase chain reactions are conducted using reverse transcribed camphor RNA as template. The reactions are conducted in a Biosycler Oven (Bios Corp.; New Haven, CT) programmed for the following cycles:

| | | | |
|---|---|---|---|
| N | 95°C for 2 min. 1 sec. drop to 65°C hold 65°C for 1 sec. 2 min. drop to 45°C hold 45°C for 30 sec. 1 sec. rise to 72°C hold 72°C for 30 sec. 1 sec. rise to 95°C | P | 95°C for 15 sec. 1 sec. drop to 65°C hold 65°C for 1 sec. 2 min. drop to 55°C hold 55°C for 15 sec. 1 sec. rise to 72°C hold 72°C for 15 sec. 1 sec. rise to 95°C |

Cycle N is run and repeated 6 times after which cycle P is run and repeated 37 times.

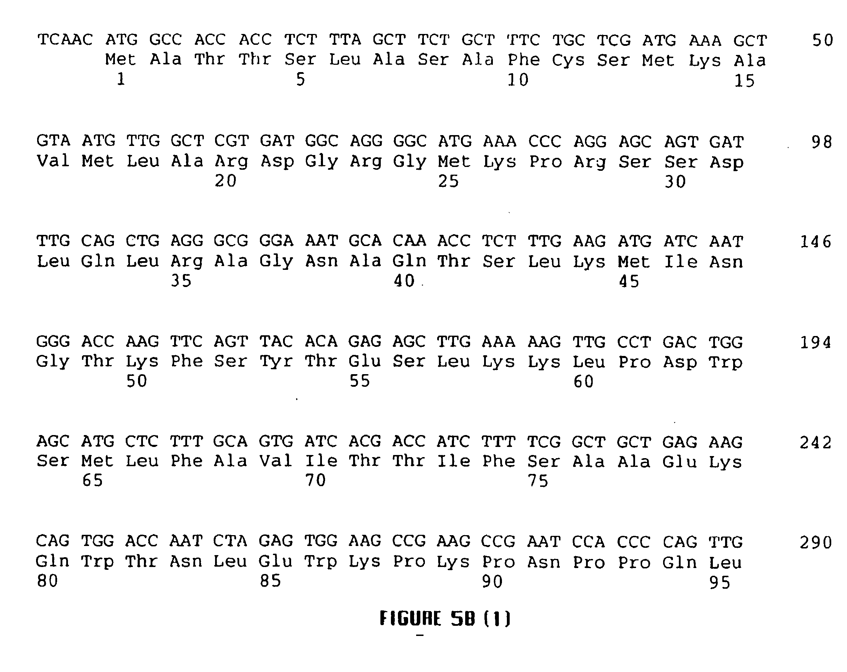

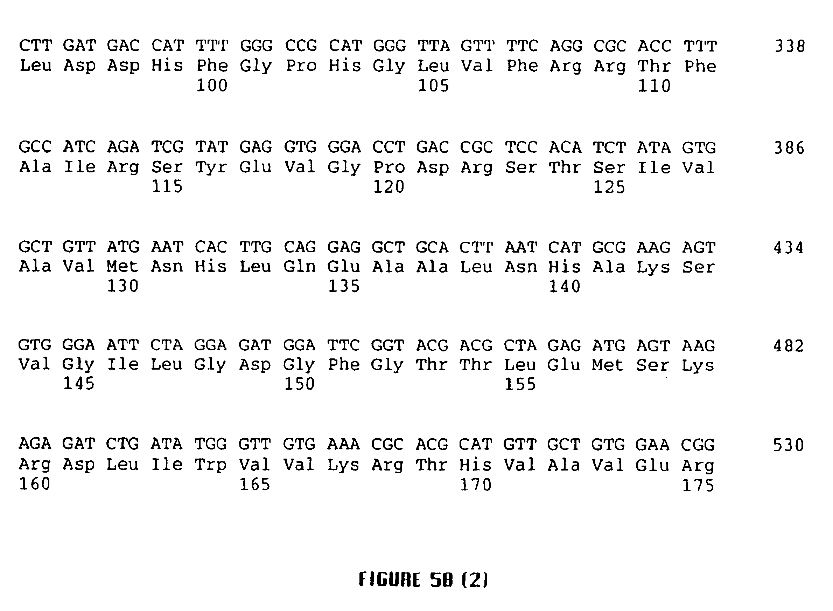

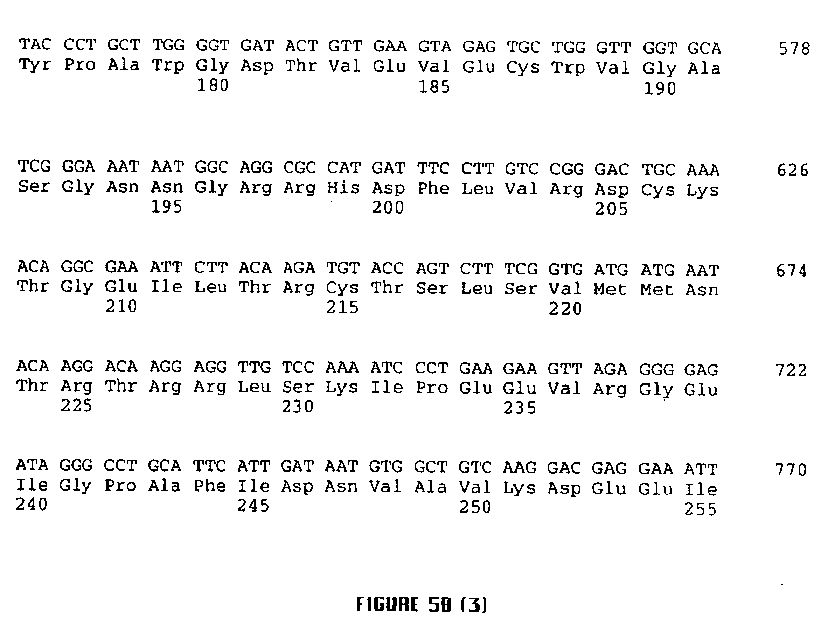

An approximately 500-600bp band is identified by agarose gel electrophoresis of the PCR products. This is the approximate fragment size predicted from analysis of the distance between the peptides in the bay thioesterase sequence. The PCR fragment is subcloned into an appropriate cloning vector and its DNA sequence determined to verify thioesterase sequence. DNA sequence of the camphor PCR fragment is presented in Figure 5A. The fragment can then be utilized to screen a camphor cDNA or genomic library to isolate a camphor thioesterase clone.

Alternative to screening gene libraries, additional PCR techniques may be used to recover entire thioesterase encoding sequences. For example, the camphor thioesterase PCR fragment sequence is used to generate additional camphor thioesterase encoding sequence. For sequences 3' to the PCR fragment, the RACE procedure of Frohman *et al*. (*Proc*. *Nat. Acad. Sci.* (1988) *85*:8998-9002) is utilized. Briefly, cDNA is generated from camphor endosperm poly(A)+ RNA using 200ng of RNA, a poly(T) oligonucleotide (with 5' restriction recognition sites for *Eco*RI, *Xho*I and *Sal*I) and reverse transcriptase. The product of this reaction is used in a PCR 3' RACE with an oligonucleotide encoding *Eco*RI, *Xho*I and *Sal*I recognition sites and an oligonucleotide representing nucleotides 443-463 of the camphor gene fragment of Figure 5A. The reaction is run in a Biosycler oven with the following program:

| | |
|---|---|
| 1 cycle at | 94°C for 40 sec. |
| | 50°C for 2 min. |
| | 72°C for 40 min. |
| 40 cycles at | 94°C for 40 sec. |
| | 50°C for 2 min. |
| | 72°C for 3 min. |

In this manner, an approximately 700bp fragment representing the 3' portion of the camphor thioesterase gene sequence is obtained.

In addition, 5' sequence of the camphor thioesterase encoding sequence may also be obtained using PCR. For this reaction, cDNA to camphor endosperm poly(A)+ RNA is generated using random hexamer oligonucleotide primers in a reverse transcription reaction essentially as described by Frohman *et al*. (*supra*). The cDNA product of this reaction is A-tailed using terminal deoxynucleotide transferase and used in PCR. Oligonucleotide primers for this reaction are MET-1-2898, which contains nucleotides 140-155 of the bay thioesterase sequence in Figure 1A and a 5' *Bam*HI recognition site, and 2356, a degenerate oligonucleotide containing a sequence complementary to nucleotides 115-126 of the camphor thioesterase gene fragment of Figure 5A. The reaction is run in a Biosycler oven with the following program:

| | |
|---|---|
| 35 cycles at | 94°C for 1 min. |
| | 55°C for 1.5 min. |
| | 72°C for 2.5 min. |

In this manner, an approximately 450bp fragment representing the 5' portion of the camphor thioesterase gene sequence is obtained.

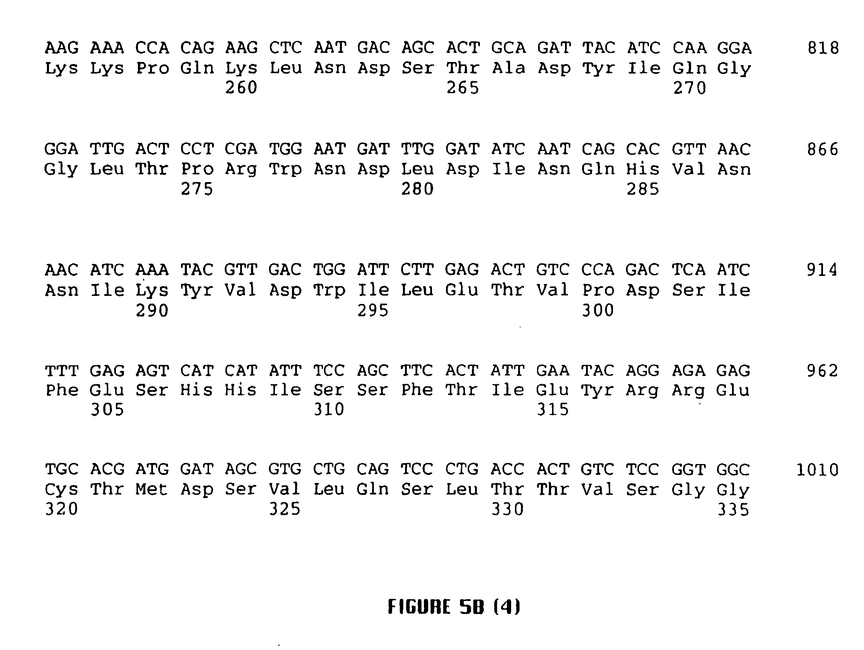

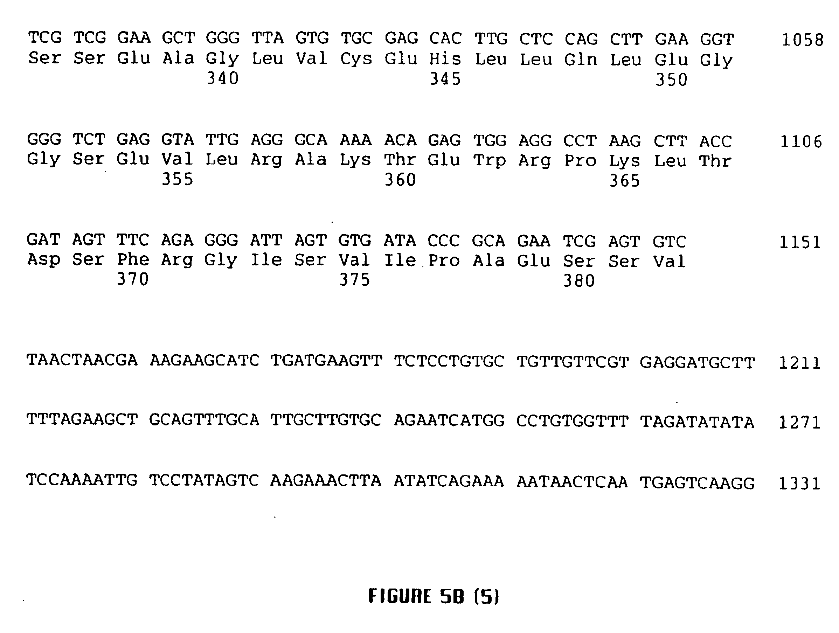

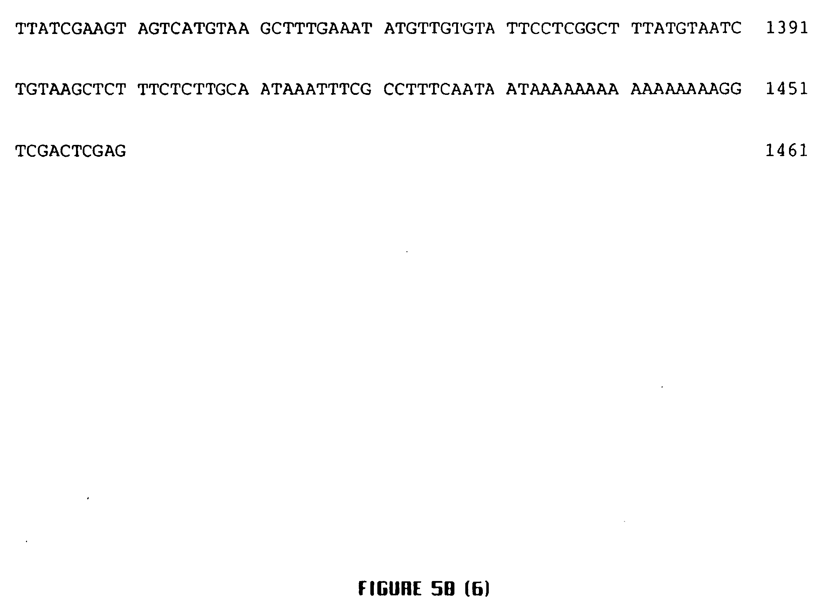

The various camphor thioesterase gene fragments are combined in a convenient cloning vector using restriction sites as inserted from the PCR procedures. Preliminary nucleic acid sequence and translated amino acid sequences of the camphor thioesterase gene generated in this manner is presented in Figure 5B.

DNA sequences corresponding to Cuphea thioesterase may also be obtained using PCR methods. Degenerate oligonucleotides for use as primers may be designed from peptide fragments that are conserved between the bay, safflower and camphor thioesterase cDNA clones. The forward primer, TECU3, contains 18 nucleotides corresponding to all possible coding sequences for amino acids 283-288 of the bay (Figure 1B) and camphor (Figure 5B) thioesterase proteins, and amino acids 282-287 of the safflower thioesterase of Figure 4A. The reverse primer, TECU4A, contains 17 nucleotides corresponding to all possible coding sequences for amino acids 315-320 of the bay (Figure 1B) and camphor (Figure 5B) thioesterase proteins, and amino acids 314-319 of the safflower thioesterase of Figure 4A. In addition, the forward and reverse primers contain *Bam*HI or *Xho*I restriction sites, respectively, at the 5' end, and an inosine nucleotide at the 3' end. Inosine residues at the 3' terminus have been reported to enhance amplification from degenerate oligonucleotide primers (Batzer *et al*. (1991) *Nucl*. *Acids Res*. *19*:5081). The safflower peptides differ from the bay and camphor sequences in one amino acid in each of the designated peptide regions, and thus the oligonucleotide primers degeneracy is such that they encode both the safflower and bay/camphor sequences.

Polymerase chain reaction samples (100µl) are prepared using reverse transcribed *Cuphea hookeriana* RNA as template and 1µM of each of the oligonucleotide primers. Samples are boiled for 5 minutes and cooled to 75°C prior to addition of Taq enzyme. PCR is conducted in a Perkin-Elmer thermocycler programmed for the following temperature cycle:
94°C for 1 min.
65°C for 1 sec.
2 min. drop to 40°C
hold 40°C for 30 sec.
1 min. rise to 72°C
1 sec. rise to 94°C
repeat cycle 40 times.
A termination cycle of 2 minutes at 72°C is then run.

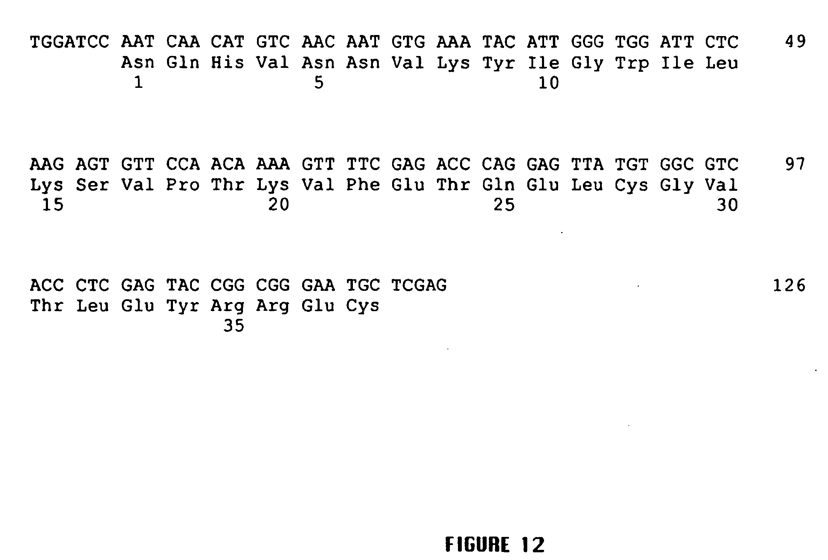

PCR products are analyzed by agarose gel electrophoresis, and an approximately 120 bp DNA fragment, the predicted size from the thioesterase peptide sequences, is observed. The DNA fragment is isolated and cloned into a convenient plasmid vector using the PCR-inserted *Bam*HI and *Xho*I restriction digest sites. The cloned fragments are sequenced, and three clones are identified which match 21 out of 38 amino acids of the corresponding bay (Figure 1B) thioesterase sequence (including the 12 amino acids encoded by the primers). Further comparison of one clone, CUPHEA-14-2, indicates that the translated peptide sequence matches 25 amino acids in the corresponding bay D (Figure 3) region, 22 in the camphor thioesterase, and 22 and 23, respectively in the safflower 2-1 and 5-2 encoded thioesterase sequences. The DNA sequence of the CUPHEA-14-2 clone and amino acid translation of the thioesterase coding region are presented in Figure 12. The thioesterase encoding fragment is labeled and used to screen a *Cuphea hookeriana* cDNA library to isolate the corresponding thioesterase cDNA.

### Analysis of Thioesterase Sequences

Clones identified using DNA hybridization or immunological screening techniques are then purified and the DNA isolated using techniques as provided in Maniatis, *et al*. (*supra*). DNA sequence of the genes is determined to verify that the clones encode a related thioesterase. Alternatively, the protein is expressed in *E*. *coli* to show that it has the desired activity. The newly isolated plant thioesterase sequences can also be used to isolate genes for thioesterases from other plant species using the techniques described above.

For example, comparison of amino acid and nucleic acid sequences of the Bay, camphor and safflower thioesterases reveals homology that is useful for isolation of additional thioesterase genes. The bay and camphor clones demonstrate extensive homology, especially at the amino acid level, and may be useful for isolation of other thioesterases having similar short or medium-chain acyl-ACP substrate specificities, such as *Cuphea*, elm, nutmeg, etc. Similarly, the long chain thioesterase genes of safflower or *Brassica,* which have significant homology, may be useful for isolation of plant thioesterases having specificities for longer chain acyl-ACP substrates, such as those identified from Chinese tallow or cotton which have specificity for 16:0 fatty-acyl ACP and mango (18:0).

In addition, regions of the long chain thioesterase proteins and the short or medium-chain specific thioesterase proteins also demonstrate homology. These homologous regions may be useful for designing degenerate oligonucleotides for use in PCR to isolate additional plant thioesterases. For example, as described above, oligonucleotides to bay and safflower thioesterase regions were used to obtain camphor thioesterase encoding sequence. This conserved region corresponds to amino acids 113-119 of the bay and camphor amino acid sequences in Figures 1B and 5B, respectively and amino acids 108-114 of the safflower amino acid sequence in Figure 4A. Similarly, other conserved regions are found in the bay, camphor and safflower amino acid sequences (as shown in Figures 1B, 5B and 4B, respectively), such as in 174-188 of bay and camphor and 169-183 of safflower; 219-229 of bay and camphor and 214-224 of safflower; and 138-145 of bay and camphor and 133-140 of safflower.

The above described plant acyl-ACP thioesterases are more highly conserved towards the center of the proteins than at either the carboxy- or amino-termini. The conserved regions may represent areas related to the catalytic site of the enzyme, and the observed substrate specificity differences may be related to the amino acid sequence differences in the regions at either end of the polypeptide chain. The plant acyl-ACP thioesterase protein sequences do not contain an active site consensus sequence (GHSxG) that is found in animal and yeast thioesterases and other fatty acid synthesis enzymes, or the active site motif of the cysteine-based hydrolases (Aitken (1990) in *Identification of Protein Consensus Sequences*, Ellis Horwood, London, pp. 81-91). As inhibitor studies indicate that the plant thioesterase enzymes are sensitive to sulfhydryl-specific reagents such as N-ethylmaleimide (Pollard, *et al*., *supra),* a cysteine residue may be involved at the active site.

Thus, other plant thioesterase genes may be isolated by the above described methods and used for expression of plant thioesterases. In particular, expression in *E. coli* will be useful for verifying the acyl chain length specificity of these thioesterases, and expression in plant seeds will be useful for producing modified oils.

### Example 6 - Plant Thioesterases and Dehydrases in Plants

The enzyme 3-hydroxydecanoyl-[acyl-carrier-protein] dehydratase (EC 4.2.1.60), also referred to herein as dehydrase, catalyzes the dehydration of 3-hydroxydecanoyl-ACP (C10:0-ACP) to 2-decenoyl-ACP (C10:1-ACP), a key step in the production of unsaturated fatty acids in bacteria. Expression of this enzyme in plant seeds is useful for production of unsaturated mdeium-chain acyl-ACPs in plants which also contain the bay medium-chain acyl-ACP thioesterase gene. In this manner, medium-chain unsaturated free fatty acids are formed as the result of hydrolysis activity of the bay thioesterase on C12:1 and C14:1 substrates.

A useful construct for expression of dehydrase in plant seeds provides for expression of the enzyme in plant seed tissue under control of a napin promoter region. In addition, a transit peptide region is provided for translocation of the dehydrase enzyme into plastids.

A dehydrase nucleic acid sequence from the *E. coli* dehydrase gene (Cronan *et al*. (1988) *J. Biol. Chem. 263*:4641-4646) is constructed, which encodes all but the initial Met amino acid of the dehydrase enzyme. A PCR DNA fragment which encodes the safflower thioesterase transit peptide and 6 amino acids of the mature safflower thioesterase (from clone 2-1) is inserted immediately 5' to the dehydrase such that the transit peptide and dehydrase sequences are in the same reading frame. The safflower thioesterase transit/dehydrase sequence is inserted into the napin expression cassette, pCGN3223, between the 5' and 3' napin regulatory sequences.

The dehydrase expression construct is transformed into a binary construct for plant transformation. A vector which encodes a selectable marker other than kanamycin is preferred. In this manner, transgenic *Brassica* plants which produce medium-chain acyl-ACP fatty acids as the result of an inserted bay thioesterase construct (such as those described in Example 4), may be re-transformed with the dehydrase expression construct. For example, the dehydrase expression construct may be inserted into a binary vector, pCGN2769 (described below), which encodes resistance to the antibiotic hygromycin B. *Agrobacterium* cells containing the resulting construct are obtained and used in *Brassica* transformation methods as described in Example 3.

The binary vector, pCGN2769, contains the right and left borders of *Agrobacterium* T-DNA, and between these borders, a 35S/hygromycin/tr7 construct for selection of transformed plant cells. The vector was constructed to be directly analogous to the binary vectors described by McBride and Summerfelt *(supra),* except for the use of an alternate selectable marker. The *hph* gene encoding hygromycin B phosphotransferase is described by Gritz and Davies (*Gene* (1983) 25:179-188). A DNA *Xho*I fragment containing the following *hph* and plant regulatory sequences was constructed using polymerase chain reaction techniques: - 289 to +114 (relative to the transcriptional start site) of a CaMV35S promoter; *hph* coding region nucleotides 211-1236 (Gritz and Davies; *supra*), with the ATG initiation codon contained in the sequence ATCATGAAA, to provide a plant concensus translation initiation sequence (Kozak (1989) *J. Cell. Biol. 108*:229-241); an *Agrobacterium* transcript 7 (tr7) transcription termination region, from nucleotides 2921-2402 of T-DNA as numbered by Barker *et al*. *(Plant Mol. Biol*. (1983) *2*:335-350). The *Xho*I *hph* expression fragment was ligated into pCGN1541 to create pCGN2768 which has a *Bgl*II fragment containing the left border of pTiA6 T-DNA, the *hph* expression construct, a *Hae*II fragment containing the 425 bp *E. coli* lac alpha encoding region, and the right border of pTiA6 T-DNA (T-DNA border and lac-α regions are described in McBride *et al*. (*supra*). The above described *Bgl*II fragment is cloned into the unique *Bam*HI fragment of pCGN1532 McBride *et al*. (supra) resulting in pCGN2769.

Alternatively, the dehydrase expression construct and a bay thioesterase expression construct (such as pCGN3828) may both be inserted into a single binary vector, such as the McBride *et al*. *(supra)* vectors which contain a marker for selection of kanamycin resistant plants. In either of these methods, plants which are able to produce medium-chain unsaturated and saturated fatty acids are produced.

All publications and patent applications mentioned in this specification are indicative of the level of skill of those skilled in the art to which this invention pertains.

Although the foregoing invention has been described in some detail by way of illustration and example for purposes of clarity of understanding, it will be obvious that certain changes and modifications may be practiced within the scope of the appended claim.

## Claims

1. A method of producing an unsaturated medium-chain free fatty acid comprising
contacting, under enzyme reactive conditions, (1) an unsaturated fatty acyl-ACP substrate and (2) a plant medium-chain thioesterase, and said plant thioesterase being obtainable by expression of a DNA structural gene encoding a Bay thioesterase having the sequence of Figure 1B and being capable of hydrolyzing a saturated fatty acyl-ACP substrate of the same length as said unsaturated fatty acyl-ACP substrate, whereby a medium-chain fatty acid is released from ACP.

2. The method of claim 1 wherein said contacting occurs as the result of the expression of said Bay thioesterase within an *E*. *coli* cell.

3. The method of claim 1 or 2 wherein at least one of C12:1 or C14:1 is produced.

4. The method of any one of claims 1 to 3 wherein said contacting occurs in a plant cell.

5. The method of claim 4 wherein said unsaturated fatty acyl-ACP substrate is produced from the steps of contacting, under enzyme reactive conditions, (a) a saturated fatty acyl-ACP substrate and (b) a β-hydroxydecanoyl thioesterase dehydrase.

6. A method of harvesting medium-chain fatty acids from a bacterial cell comprising
culturing a bacterial cell having a DNA sequence encoding a plant medium-chain thioesterase encoding a Bay thioesterase having the sequence of Figure 1B, under the control of regulatory sequences functional in said cell under conditions to result in the expression of said thioesterase, wherein said cell in deficient in fatty acid degredation; and recovering fatty acid salts from a cell free medium.

7. The method of claim 6 wherein said bacterial cell is acyl-CoA synthase deficient and selected from the group consisting of *E*. *coli fad*D and *E*. *coli fad*E.

8. The method of claim 6 or 7 wherein said bacterial cell is cultured at a temperature of about 25-30°C.

9. The method of any one of claims 6 to 8 wherein said fatty acid salts are extracellularly deposited laurate salt crystals.

10. The method of claim 9 wherein said fatty acid salts are unsaturated fatty acids.

11. A plant seed comprising a minimum of 1.0 mole percent laurate in total fatty acids, wherein said laurate is incorporated into at least one position of a triglyceride molecule, the plant seed containing a heterologous DNA structural gene encoding a Bay thioesterase having the sequence of Figure 1B, and wherein wild-type seed of said plant contains less than 1.0 mole percent laurate in fatty acids.

12. The seed of claim 11 comprising a minimum of about 15 mole percent laurate in fatty acids.

13. The seed of claim 11 comprising a minimum of about 50 mole percent laurate in fatty acids.

14. A seed according to any one of claims 11 to 13 which is a *Brassica* seed.

15. A DNA construct capable of producing a plant thioesterase in a host cell comprising, in the 5' to 3' direction of transcription, a transcriptional initiation region functional in said host cell, a translational initiation region functional in said host cell, a DNA structural gene encoding a Bay thioesterase having the sequence of Figure 1B, and a transcriptional and translational termination region functional in said host cell.

## Patentansprüche

1. Verfahren zur Herstellung einer ungesättigten, mittelkettigen, freien Fettsäure, umfassend:
das Inkontaktbringen von (1) eines ungesättigten Fettacyl-ACP-Substrats und von (2) einer pflanzlichen mittelkettigen Thioesterase unter Enzymreaktionsbedingungen, und
wobei die pflanzliche Thioesterase erhältlich ist durch Expression eines DNA-Strukturgens, welches eine Bay-Thioesterase mit der Sequenz von Figur 1B codiert, und fähig ist, ein gesättigtes Fettacyl-ACP-Substrat mit der gleichen Länge wie das ungesättigte Fettacyl-ACP-Substrat zu hydrolysieren, wodurch eine mittelkettige Fettsäure aus ACP freigesetzt wird.

2. Verfahren nach Anspruch 1, wobei das Inkontaktbringen als Ergebnis der Expression der Bay-Thioesterase innerhalb einer E. coli-Zelle erfolgt.

3. Verfahren nach Anspruch 1 oder 2, wobei mindestens eines von C12:1 oder C14:1 hergestellt wird.

4. Verfahren nach irgendeinem der Ansprüche 1 bis 3, wobei das Inkontaktbringen in einer Pflanzenzelle erfolgt.

5. Verfahren nach Anspruch 4, wobei das ungesättigte Fettacyl-ACP-Substrat durch die Schritte des Inkontaktbringens von (a) eines gesättigten Fettacyl-ACP-Substrats und von (b) einer β-Hydroxydecanoyl-Thioesterasedehydrase unter Enzymreaktionsbedingungen hergestellt wird.

6. Verfahren zur Gewinnung von mittelkettigen Fettsäuren aus einer Bakterienzelle, umfassend:
das Züchten einer Bakterienzelle mit einer DNA-Sequenz, codierend eine pflanzliche mittelkettige Thioesterase, welche eine Bay-Thioesterase mit der Sequenz von Figur 1B codiert, unter der Kontrolle von regulatorischen Sequenzen, die in der Zelle unter Bedingungen funktional sind, welche in der Expression der Thioesterase resultieren, wobei die Zelle bezüglich des Fettsäureabbaus defizient ist; und das Gewinnen von Fettsäuresalzen aus einem zellfreien Medium.

7. Verfahren nach Anspruch 6, wobei die Bakterienzelle Acyl-CoA-Synthetasedefizient ist und gewählt ist aus der Gruppe, bestehend aus *E. coli fad*D und *E. coli fad*E.

8. Verfahren nach Anspruch 6 oder 7, wobei die Bakterienzelle bei einer Temperatur von etwa 25-30°C gezüchtet wird.

9. Verfahren nach irgendeinem der Ansprüche 6 bis 8, wobei die Fettsäuresalze extrazellulär abgelagerte Lauratsalzkristalle sind.

10. Verfahren nach Anspruch 9, wobei die Fettsäuresalze ungesättigte Fettsäuren sind.

11. Pflanzensamen, umfassend mindestens 1,0 Molprozent Laurat in den Fettsäuren insgesamt, wobei das Laurat in mindestens eine Position eines Triglyceridmoleküls eingebaut ist; wobei die Pflanzensamen ein heterologes DNA-Strukturgen enthalten, das eine Bay-Thioesterase mit der Sequenz von Figur 1B codiert, und wobei die Wildtyp-Samen der Pflanze weniger als 1,0 Molprozent Laurat in den Fettsäuren enthalten.

12. Samen nach Anspruch 11, umfassend mindestens etwa 15 Molprozent Laurat in den Fettsäuren.

13. Samen nach Anspruch 11, umfassend mindestens etwa 50 Molprozent Laurat in den Fettsäuren.

14. Samen nach irgendeinem der Ansprüche 11 bis 13, welche *Brassica*-Samen sind.

15. DNA-Kontrukt, welches fähig ist, eine pflanzliche Thioesterase in einer Wirtszelle herzustellen, umfassend, in der 5'-3'-Transkriptionsrichtung, eine in der Wirtszelle funktionale Transkriptionsinitiationsregion, eine in der Wirtszelle funktionale Translationsinitiationsregion, ein DNA-Strukturgen, codierend eine Bay-Thioesterase mit der Sequenz von Figur 1B, und eine Transkriptions- und Translationsterminationsregion, welche in der Wirtszelle funktional ist.

## Revendications

1. Procédé de production d'un acide gras libre à chaîne moyenne insaturée comprenant
la mise en contact, dans des conditions réactionnelles enzymatiques, (1) d'un substrat acyl-ACP gras insaturé et (2) d'une thioestérase à chaîne moyenne d'origine végétale, ladite thioestérase d'origine végétale pouvant être obtenue par expression d'un gène structural d'ADN codant pour une thioestérase Bay ayant la séquence de la figure 1B et étant susceptible d'hydrolyser un substrat acyl-ACP gras saturé de la même longueur que ledit substrat acyl-ACP gras insaturé, par laquelle un acide gras à chaîne moyenne est libéré d'ACP.

2. Procédé selon la revendication 1 dans lequel ladite mise en contact se présente comme le résultat de l'expression de ladite thioestérase Bay à l'intérieur d'une cellule de *E*. *coli*.

3. Procédé selon la revendication 1 ou 2 dans lequel l'un au moins de C12:1 ou C14:1 est produit.

4. Procédé selon l'une quelconque des revendications 1 à 3 dans lequel ladite mise en contact a lieu dans une cellule végétale.

5. Procédé selon la revendication 4 dans lequel ledit substrat acyl-ACP gras insaturé est produit à partir de l'étape de mise en contact, dans des conditions réactionnelles enzymatiques, (a) d'un substrat acyl-ACP gras saturé et (b) d'une β-hydroxydécanoyle thioestérase déhydrase.

6. Procédé de récolte d'acides gras à chaîne moyenne à partir d'une cellule bactérienne comprenant
la culture d'une cellule bactérienne ayant une séquence d'ADN codant pour une thioestérase à chaîne moyenne d'origine végétale codant pour une thioestérase Bay ayant la séquence de la figure 1B, sous le contrôle de séquences régulatrices fonctionnelles dans ladite cellule, dans des conditions qui résultent en l'expression de ladite thioestérase, ladite cellule étant déficiente en dégradation des acides gras ; et
la récupération de sels d'acides gras à partir d'un milieu acellulaire.

7. Procédé selon la revendication 6 dans lequel ladite cellule bactérienne est déficiente en acyl-CoA synthase et choisie dans le groupe constitué par *E. coli fad*D et *E*. *coli fad*E.

8. Procédé selon la revendication 6 ou 7 dans lequel ladite cellule bactérienne est cultivée à une température d'environ 25-30°C.

9. Procédé selon l'une quelconque des revendications 6 à 8 dans lequel lesdits sels d'acides gras sont des cristaux de sel de laurate déposés extracellulairement.

10. Procédé selon la revendication 9 dans lequel lesdits sels d'acides gras sont des acides gras insaturés.

11. Graine végétale comprenant un minimum de 1,0 pour cent en mole de laurate en acides gras totaux, dans laquelle ledit laurate est incorporé sur au moins une position d'une molécule de triglycéride, la graine végétale contenant un gène structural d'ADN hétérologue codant pour une thioestérase Bay ayant la séquence de la figure 1B, et une graine de type sauvage de ladite plante contenant moins de 1,0 pour cent en mole de laurate en acides gras.

12. Graine selon la revendication 11 comprenant un minimum d'environ 15 pour cent en mole de laurate en acides gras.

13. Graine selon la revendication 11 comprenant un minimum d'environ 50 pour cent en mole de laurate en acides gras.

14. Graine selon l'une quelconque des revendications 11 à 13 qui est une graine de *Brassica.*

15. Construction d'ADN susceptible de produire une thioestérase d'origine végétale dans une cellule hôte comprenant, dans la direction 5' à 3' de la transcription, une région d'initiation transcriptionnelle fonctionnelle dans ladite cellule hôte, une région d'initiation traductionnelle fonctionnelle dans ladite cellule hôte, un gène structural d'ADN codant pour une thioestérase Bay ayant la séquence de la figure 1B, et une région de terminaison transcriptionnelle et traductionnelle fonctionnelle dans ladite cellule hôte.
